(19) Europäisches Patentamt
European Patent Office
Office européen des brevets

(11) Numéro de publication: **0 449 034 B1**

(12) # FASCICULE DE BREVET EUROPEEN

(45) Date de publication de fascicule du brevet: **28.12.94**

(21) Numéro de dépôt: **91103803.2**

(22) Date de dépôt: **13.03.91**

(51) Int. Cl.5: **C07C 49/11**, C07C 49/21, C07C 45/72, C07C 45/73, C07C 47/225, C07D 303/04, A61K 7/46

(54) **Cétones cycliques nouvelles, procédé pour leur préparation et leur utilisation en parfumerie.**

(30) Priorité: **26.03.90 CH 997/90**

(43) Date de publication de la demande:
**02.10.91 Bulletin 91/40**

(45) Mention de la délivrance du brevet:
**28.12.94 Bulletin 94/52**

(84) Etats contractants désignés:
**CH DE FR GB LI NL**

(56) Documents cités:
**EP-A- 0 075 237**
**EP-A- 0 121 828**
**EP-A- 0 374 509**
**FR-A- 2 404 616**

**"Chemical Abstracts Registry Handbook [1965-1971] [R1] 1R-2024R" 1974, American Chemical Society, Columbus, Ohio, US**

(73) Titulaire: **FIRMENICH SA**
**1, route des Jeunes**
**CH-1211 Genève 8 (CH)**

(72) Inventeur: **Chapuis, Christian**
**5, Vy de Montorge**
**CH-1295 Mies (CH)**
Inventeur: **Schulte-Elte, Karl-Heinrich**
**44, chemin Carabot**
**CH-1213 Onex (CH)**
Inventeur: **Pamingle, Hervé**
**3, chemin Ami-Argand**
**CH-1290 Versoix (CH)**
Inventeur: **Margot, Christian**
**Chemin de la Ravière**
**CH-1261 Bassins (CH)**

(74) Mandataire: **Salvadori, Giuseppe, Dr.**
**c/o Firmenich S.A.**
**Case Postale 239**
**CH-1211 Genève 8 (CH)**

**EP 0 449 034 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

**HELVETICA CHIMICA ACTA. vol. 72, no. 6, 20 septembre 1989, BASEL CH pages 1400 - 1415; B. Maurer et al.: "New Irone-Related Constituents from the Essential Oil of Iris Germanica L."**

**PATENT ABSTRACTS OF JAPAN vol. 11, no. 242 (C-438)(2689) 07 août 1987, & JP-A-62 051637 (T. HASEGAWA) 06 mars 1987,**

**PATENT ABSTRACTS OF JAPAN vol. 11, no. 89 (C-411)(2536) 19 mars 1987, & JP-A-61 243041 (T. HASEGAWA) 29 octobre 1986,**

## Description

La présente invention a trait au domaine de la parfumerie et de la synthèse de composés odorants, en particulier de cétones cycliques nouvelles utiles pour la préparation de compositions parfumantes et d'articles parfumés.

Le brevet européen n° 121 828, publié le 19.11.87, décrit un procédé permettant de préparer des cétones cycliques de formule

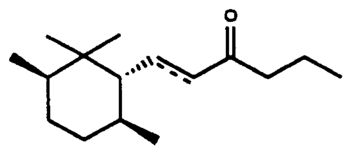

dans laquelle la ligne pointillée indique l'emplacement d'une liaison simple ou double. Ces cétones sont citées à titre de produits intermédiaires utiles pour la préparation de l'alcool saturé correspondant, intéressant la parfumerie, et le procédé décrit peut être illustré comme suit :

SCHEMA I

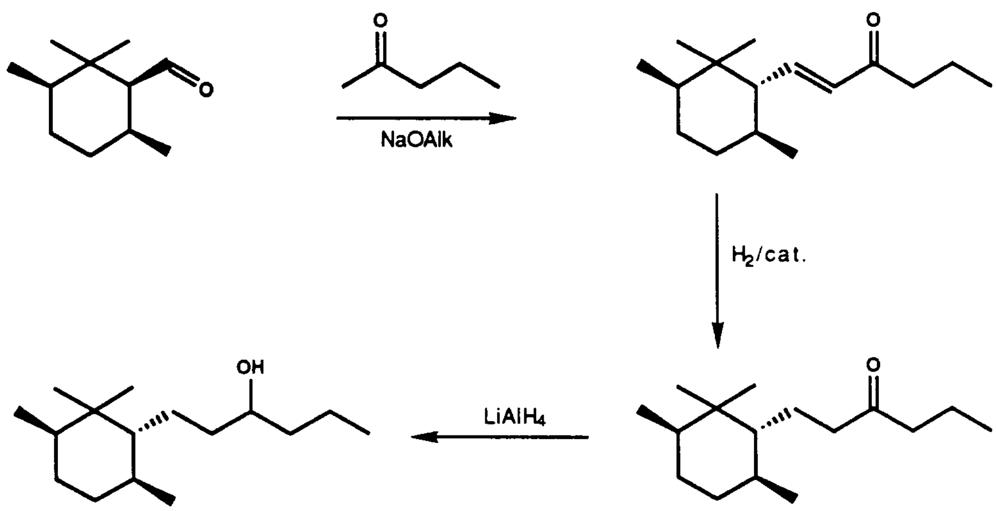


Selon le même document, le 2,2,3,6-tétraméthyl-1-cyclohexanecarbaldéhyde, utilisé comme produit de départ dans ce procédé, pouvait être obtenu à partir d'un ester dérivé de l'acide α-cyclogéranique, suivant une méthode qui peut être illustrée comme suit :

SCHEMA II

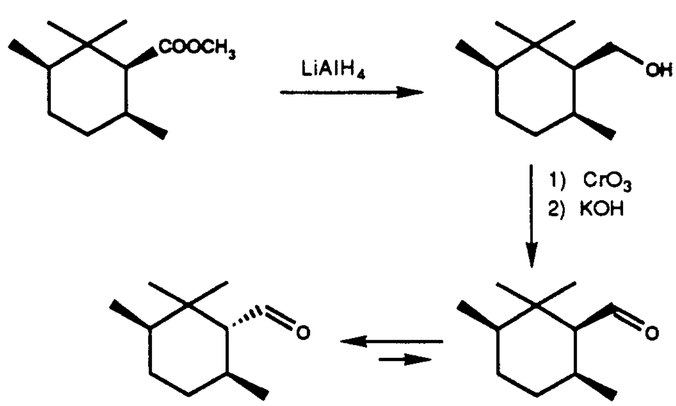

L'importance capitale du carbaldéhyde susmentionné en tant que produit intermédiaire pour la préparation de cétones et alcools odorants est citée dans le brevet mentionné.

D'autre part, la demande de brevet européen n° 0374509, publiée le 27.06.90 et portant la priorité suisse du 21.12.88, a apporté une autre contribution dans ce domaine en décrivant une méthode qui permet de préparer des formes isomériques spécifiques plus avantageuses du carbaldéhyde susmentionné, lesquelles ne pouvaient pas auparavant être obtenues avec un rendement utile. Le procédé décrit dans cette demande de brevet est illustré par le schéma suivant, dans lequel la ligne ondulée sert à désigner une liaison C-C de configuration cis ou trans et les deux substituants méthyle en positions 3 et 6 du cycle hexanique possèdent une configuration trans :

SCHEMA III

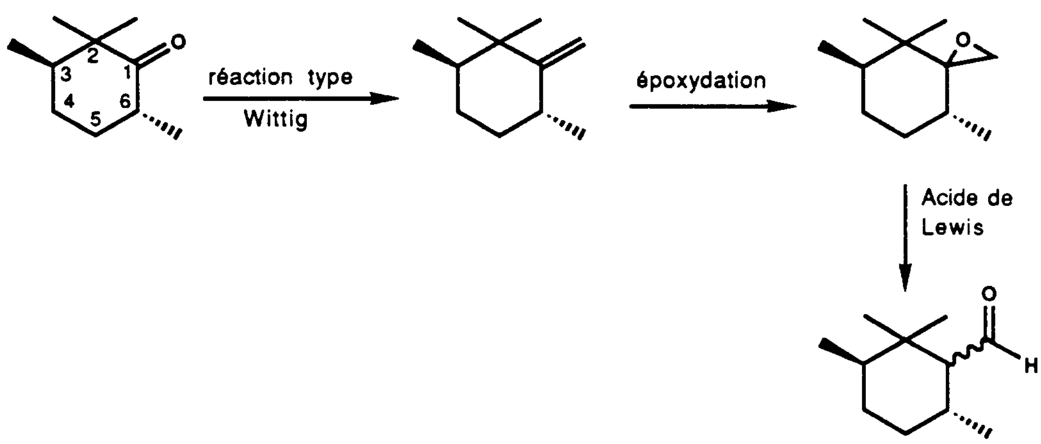


Le but des deux procédés illustrés aux schémas I et III et décrits dans les deux documents précités était donc de permettre la préparation des isomères de configuration trans du 1-(2,2,3,6-tétraméthyl-1-cyclohexyl)-3-hexanol, lesquels intéressaient particulièrement la parfumerie, la 1-(2,2,3,6-tétraméthyl-1-cyclohexyl)-1-hexén-3-one et la 1-(2,2,3,6-tétraméthyl-1-cyclohexyl)-3-hexanone étant des produits intermédiaires dans le procédé global.

Nous avons maintenant découvert que le principe sur lequel reposent ces procédés peut être appliqué à la synthèse de produits odorants nouveaux et qu'il fournit, par ailleurs, une méthode nouvelle de préparation des $\alpha$-, $\beta$- et $\gamma$-irones et ionones, composés bien connus et appréciés en parfumerie, ainsi que de leurs homologues. Les rapports ayant trait à des méthodes de synthèse de ces composés sont très nombreux à ce jour [voir, par exemple, P.Z.Bedoukian, Perfumery and Flavoring Synthetics, Allured Publishing Corporation, 3rd ed., USA (1986)], mais la préparation des irones en particulier reste très coûteuse, et des efforts continuent à être déployés dans le but de trouver des synthèses plus simples et économiques. La présente invention apporte justement une solution originale et avantageuse à ce problème et, notamment, à celui de la synthèse de formes stéréoisomères spécifiques, racémiques ou optiquement actives, de ces composés. Elle fournit par ailleurs de nouvelles cétones dont on a découvert de façon inattendue qu'elles possédaient des propriétés odorantes fort intéressantes.

Un objet de la présente invention est un procédé pour la préparation d'un composé de formule

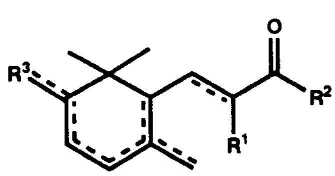

( I )

dans laquelle les lignes pointillées indiquent l'emplacement d'une liaison simple ou double, le symbole $R^1$ représente un atome d'hydrogène ou un radical méthyle, le symbole $R^2$ représente un radical alkyle de $C_1$ à $C_4$, saturé ou insaturé, de chaîne linéaire ou ramifiée et le symbole $R^3$ représente un radical méthyle, éthyle ou méthylène, à l'exclusion de la 4-(2,2,3,6-triméthyl-1-cyclohexyl)-2-butanone, la 4-(2,2,3,6-tétraméthyl-1-cyclohexyl)-3-butén-2-one, la 1-(2,2,3,6-tétraméthyl-1-cyclohexyl)-1-hexén-3-one et de la 1-(2,2,3,6-tétraméthyl-1-cyclohexyl)-3-hexanone, le procédé étant caractérisé en ce qu'on :

a. traite un composé de formule

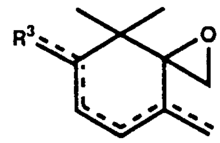

(II)

dans laquelle les lignes pointillées et le symbole $R^3$ sont définis comme ci-dessus, avec un acide de Lewis pour obtenir un aldéhyde de formule

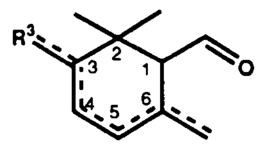

(III)

dans laquelle les lignes pointillées et le symbole $R^3$ ont le sens indiqué plus haut ;

b. transforme, le cas échéant, à l'aide d'une réaction d'isomérisation connue en soi, un aldéhyde de formule (III), possédant une double liaison endocyclique en position 5, en son isomère de formule

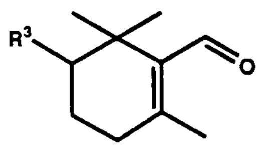

(IIIb)

dans laquelle le symbole $R^3$ a le sens indiqué plus haut ;

c. transforme, à l'aide d'une réaction de condensation aldolique avec une cétone appropriée de formule

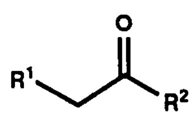

dans laquelle $R^1$ et $R^2$ sont définis comme à la formule (I), l'aldéhyde obtenu selon a. ou b. en un composé de formule (I) possédant une double liaison dans la chaîne latérale ; et,

d. soumet, le cas échéant, ce dernier composé à une réaction d'hydrogénation connue en soi, pour obtenir un composé de formule (I) possédant une chaîne saturée.

Comme cité précédemment, le procédé selon l'invention permet d'obtenir des cétones nouvelles possédant des propriétés odorantes utiles et inattendues. Il apporte en outre une solution avantageuse au problème de la synthèse de composés odorants connus et très appréciés en parfumerie, tels les irones et leurs homologues. En effet, l'étape a. de ce procédé, qui fait également l'objet de la présente invention, permet d'obtenir les aldéhydes précurseurs de ces composés et ceci à partir de produits de départ nouveaux, à savoir les époxydes (II) définis ci-dessus, à l'exclusion du 4,4,5,8-tétraméthyl-1-oxaspiro[2.5]-octane. Ce dernier est en effet décrit dans la demande de brevet européen n° 0374509 citée précédemment.

Ces époxydes nouveaux de formule (II) peuvent être préparés selon un procédé original à partir de cétones de formule

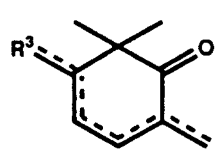

(V)

possédant une double liaison dans l'une des positions indiquées par les lignes pointillées et dans laquelle le symbole $R^3$ a le sens indiqué plus haut, suivant une méthode analogue à celle représentée au Schéma III. Alternativement, ils peuvent être préparés selon des méthodes nouvelles analogues à celles également décrites dans la demande de brevet européen déjà citée, dont le contenu est inclu ici par référence, lesquelles méthodes font appel à des réactions du type de Corey-Chaykovsky. Les conditions dans lesquelles les méthodes susmentionnées sont appliquées sont décrites en détail, cas par cas, dans les exemples de préparation des époxydes (II) présentés plus loin.

Comme il a été cité précédemment, les époxydes nouveaux de formule (II) ainsi obtenus sont, à l'instar des aldéhydes de formule (III) qui en dérivent, des composés d'une grande importance dans l'industrie des parfums, en tant que précurseurs des irones et composés analogues, et ils font également l'objet de la présente invention.

Il y a lieu de remarquer que le procédé de l'invention permet aussi la préparation d'ionones ($\alpha$,$\beta$,$\gamma$) et de leur homologues à partir d'époxydes de formule

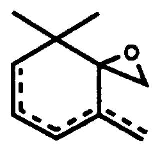

dans laquelle les lignes pointillées indiquent l'emplacement d'une liaison simple ou double. Plusieurs de ces époxydes sont des composés dont la structure est connue. Des exemples de leur préparation, ainsi que de celle des aldéhydes correspondants, sont décrits plus loin.

Selon l'invention, les époxydes de formule (II) sont transformés en aldéhydes de formule (III) à l'aide d'une réaction avec un acide de Lewis. Il s'agit de réactions analogues à celles décrites dans la demande de brevet européen n° 0374509 et les informations données à cet égard sont inclues ici par référence. Les conditions spécifiques de ces réactions sont d'ailleurs décrites, cas par cas, dans les exemples de préparation correspondants.

Le cas échéant, les aldéhydes de formule (III) qui possèdent une double liaison endocyclique en position 5 sont transformés, à l'aide de réactions d'isomérisation connues en soi, en leurs isomères de formule

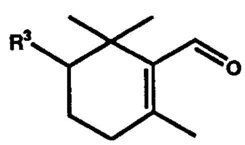

(IIIb)

dans laquelle le symbole $R^3$ est défini comme à la formule (III), qui sont des précurseurs utiles dans la préparation de $\beta$-irones et leurs analogues. Bien entendu, d'une façon analogue, des aldéhydes de formule (IIIb) dans laquelle le symbole $R^3$ est l'hydrogène, qui sont des précurseurs de $\beta$-ionones, peuvent être obtenus à partir de leurs isomères de position correspondants. Les conditions spécifiques des réactions d'isomérisation citées sont décrites plus loin.

Plusieurs aldéhydes de formule (III) ou (IIIb) sont connus à ce jour [voir, par exemple, B. Maurer et al., Helv. Chim. Acta 72, 1400 (1989); EP-A-0 075 237; JP-A-62 051 637; JP-A-61 243 041 et FR-A-2 404 616]. Cependant, les composés de formule

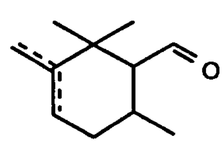

sont des composés nouveaux.

Selon le procédé de l'invention, les aldéhydes de formule (III) ou (IIIb) sont transformés en les composés de formule (I) possédant une double liaison dans la chaîne latérale, à l'aide de réactions de condensation aldolique avec une cétone appropriée de formule

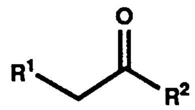

dans laquelle $R^1$ et $R^2$ sont définis comme à la formule (I) et, le cas échéant, ces composés (I) sont ensuite transformés en leurs analogues de chaîne saturée à l'aide de réactions d'hydrogénation ou réduction conventionnelles.

Selon un mode d'exécution préférentiel du procédé de l'invention, on prépare un composé de formule

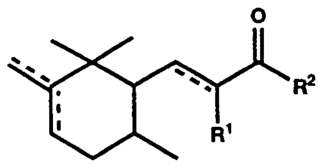

(Ia)

dans laquelle les lignes pointillées indiquent l'emplacement d'une liaison simple ou double, le symbole $R^1$ représente un atome d'hydrogène ou un radical méthyle, le symbole $R^2$ représente un radical alkyle de $C_1$ à $C_4$, saturé ou insaturé, de chaîne linéaire ou ramifiée, à l'exclusion de la 4-(2,2,3,6-tétraméthyl-1-cyclohexyl)-2-butanone, la 4-(2,2,3,6-tétraméthyl-1-cyclohexyl)-3-butén-2-one, la 1-(2,2,3,6-tétraméthyl-1-cyclohexyl)-1-hexén-3-one et de la 1-(2,2,3,6-tétraméthyl-1-cyclohexyl)-3-hexanone, en utilisant, en tant que produit de départ, un époxyde de formule

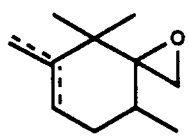

(IIa)

dans laquelle les lignes pointillées ont le sens indiqué à la formule (Ia).

D'autres modes d'exécution particulièrement préférés du procédé selon l'invention permettent de préparer des isomères spécifiques des composés de formule (I) ou (Ia) à partir des isomères appropriés des époxydes de formule (II) ou (IIa).

C'est ainsi que l'on prépare un composé essentiellement sous une forme isomérique trans de formule

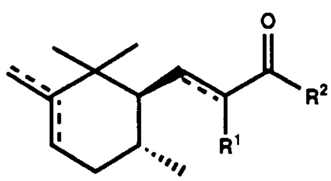

(Ib)

dans laquelle les lignes pointillées dans le cycle indiquent l'emplacement d'une liaison double, la ligne pointillée dans la chaîne latérale indique l'emplacement d'une liaison simple ou double, le symbole $R^1$ représente un atome d'hydrogène ou un radical méthyle et le symbole $R^2$ représente un radical alkyle de $C_1$ à $C_4$, saturé ou insaturé, de chaîne linéaire ou ramifiée, sous forme racémique ou de l'un des énantiomères optiquement actifs, en utilisant comme produit de départ un époxyde essentiellement sous une forme isomérique trans de formule

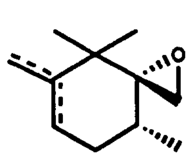

(IIb)

dans laquelle les lignes pointillées sont définies comme à la formule (Ib), ou l'un de ses énantiomères optiquement actifs,

et que l'on prépare un composé essentiellement sous une forme isomérique trans de formule

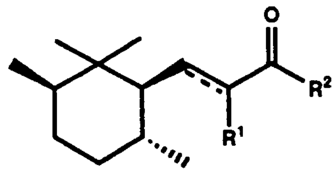

(Ic)

dans laquelle la ligne pointillée indique l'emplacement d'une liaison simple ou double et les symboles $R^1$ et $R^2$ ont le sens indiqué à la revendication 1, sous forme d'un racémate ou de l'un des énantiomères optiquement actifs, à l'exclusion de la 4-(2,2,c-3,t-6-tétraméthyl-r-1-cyclohexyl)-2-butanone, de la 1-(2,2,c-3,t-6-tétraméthyl-r-1-cyclohexyl)-1-hexén-3-one et de la 1-(2,2,c-3,t-6-tétraméthyl-r-1-cyclohexyl)-3-hexanone, en utilisant comme produit de départ un époxyde essentiellement sous une forme isomérique trans de formule

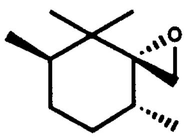

(IIc)

ou l'un de ses énantiomères optiquement actifs.

Selon l'invention, les époxydes de formule (IIa) définie plus haut, qui permettent d'obtenir des aldéhydes de formule

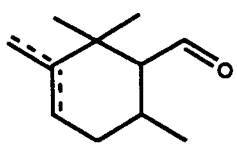

(IIIa)

dans laquelle les lignes pointillées indiquent l'emplacement d'une liaison double et, plus particulièrement, les formes stéréoisomères, racémiques ou optiquement actives de formule (IIb) ou (IIc), ou encore de formule

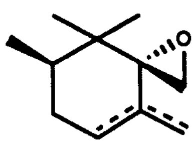

(IId)

dans laquelle les lignes pointillées indiquent l'emplacement d'une liaison double, sont des produits de départ préférés, spécifiquement adaptés à la préparation de stéréoisomères préférés des composés (I) dont les propriétés sont particulièrement intéressantes, comme il ressort des remarques présentées plus loin.

Comme il est illustré au schéma III, ces différentes formes stéréoisomères des époxydes (II) peuvent être obtenues en utilisant comme produit de départ une forme stéréoisomère adéquate de la cétone (V). De même, des mélanges racémiques ou des isomères optiquement actifs des époxydes désirés peuvent être préparés à partir de mélanges racémiques, ou respectivement d'isomères optiquement actifs, de la cétone de départ, comme il est décrit dans les exemples de préparation présentés plus loin.

Les époxydes préférés susmentionnés sont transformés, selon le procédé de l'invention, dans les aldéhydes correspondants, parmi lesquels les aldéhydes sous une forme stéréoisomère racémique de formule

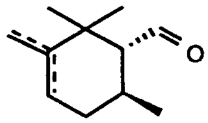

(IIIc)

dans laquelle les lignes pointillées indiquent l'emplacement d'une liaison double, ou sous forme de l'un des énantiomères optiquement actifs, sont cités à titre préférentiel.

Les composés (I) de l'invention peuvent également être préparés selon un procédé alternatif, dans lequel l'aldéhyde (III) est obtenu par une série de réactions du type de celles illustrées au Schéma II. Nous avons constaté que ce procédé convenait particulièrement bien à la préparation des composés de formule (Ia) qui possédaient une double liaison dans l'une des positions du cycle indiquées par les lignes pointillées, et qu'il pouvait être utilisé avantageusement pour la synthèse des isomères de configuration trans de formule (Ib). Ce procédé, dont des exemples spécifiques sont présentés plus loin, fait usage d'une réaction de réduction d'un ester de départ de formule

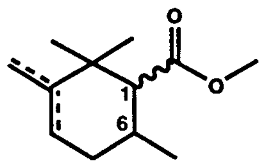

(VI)

dans laquelle les lignes pointillées ont le sens indiqué ci-dessus et la ligne ondulée désigne une liaison C-C de configuration cis ou trans par rapport au groupe méthyle substituant en position 6, à l'aide d'un aluminohydrure de métal alcalin pour obtenir un alcool de formule

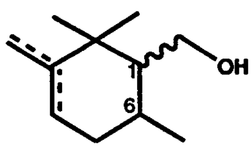

(VII)

dans laquelle les lignes pointillées et la ligne ondulée ont le sens indiqué à la formule (VI), réaction qui est suivie d'un traitement dudit alcool avec un agent oxydant pour obtenir un aldéhyde de formule

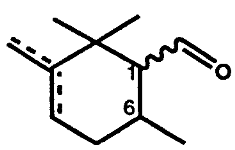

(IIIa)

dans laquelle les lignes pointillées et la ligne ondulée ont le sens indiqué.

La réduction de l'ester de formule (VI) peut être réalisée par exemple à l'aide de $LiAlH_4$ et, en tant qu'agent oxydant de l'alcool (VII) ainsi obtenu, on peut utiliser notamment le chlorochromate de pyridinium (PCC) dans le dichlorométhane. L'aldéhyde de formule (IIIa) résultant de cette oxydation est ensuite transformé en le composé (Ib) désiré à l'aide de réactions du type de celles représentées au schéma I, à savoir, condensation aldolique suivie, le cas échéant, d'une hydrogénation.

Les conditions spécifiques des réactions mentionnées ci-dessus sont décrites en détail dans les exemples de préparation présentés plus loin.

Les esters de formule (VI) utilisés comme produits de départ dans ce procédé sont des composés nouveaux. Ils peuvent être préparés à partir d'un mélange d'isomères du géraniate de méthyle, suivant le schéma réactionnel que voici :

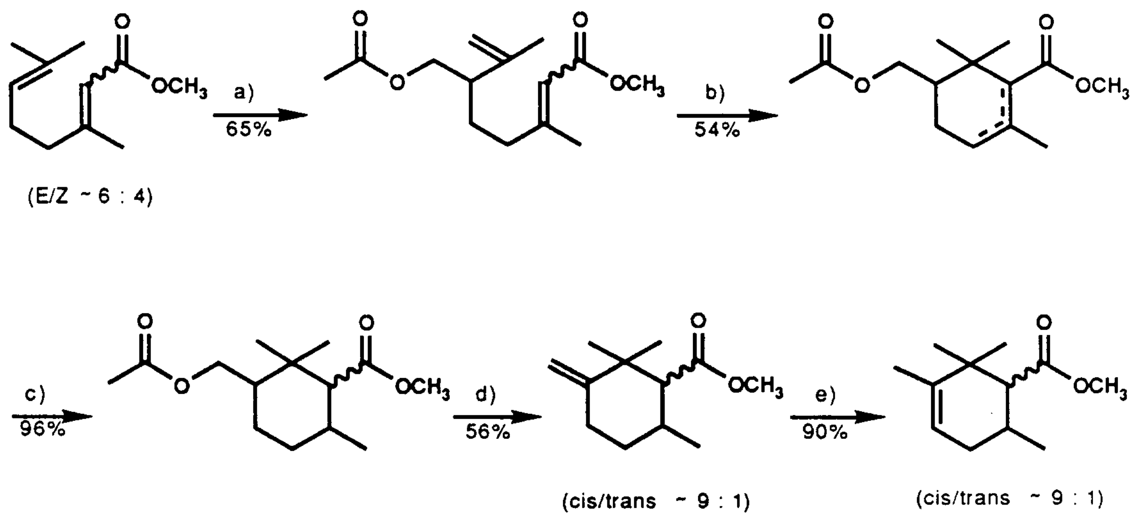


a) $CH_2O$ ; $(CH_3CO)_2O$ ; $BF_3.Et_2O$

b) $CH_3COOH$ ; $SnCl_4$ ;

c) $H_2$ ; $PtO_2$ ; $CH_3COOH$ ;

d) 430° ; $N_2$ ;

e) TsOH ; toluène

Comme il est indiqué dans ce schéma, les esters (VI) sont obtenus sous forme d'un mélange d'isomères riche en l'isomère cis. Le rapport isomérique indiqué reste sensiblement le même lors de la transformation de ces esters en alcools (VII) et, par la suite, de ces derniers en aldéhydes (IIIa). La condensation aldolique de ces aldéhydes est accompagnée d'une épimérisation plus ou moins complète pour fournir la cétone désirée sous sa forme trans (Ib).

Parmi les composés de formule (Ia) selon la présente invention, il en est dont la structure chimique est connue. Par exemple, les 1-(2,2,3,6-tétraméthyl-1-cyclohexyl)-1-hexén-3-one et 1-(2,2,3,6-tétraméthyl-1-cyclohexyl)-3-hexanone ont été décrits dans EP 121 828 à titre de produits intermédiaires dans la synthèse du 1-(2,2,3,6-tétraméthyl-1-cyclohexyl)-3-hexanol. Néanmoins, nous n'avons trouvé aucune mention dans ce brevet d'un éventuel intérêt, du point de vue olfactif, des deux cétones citées plus haut.

D'autre part, quoique la 4-(2,2,3,6-tétraméthyl-3-cyclohexén-1-yl)-3-butén-2-one soit citée dans le Chemical Abstracts Registry Handbook (1965-1971), page 243 R, colonne 2, ligne 32, nous n'avons pu trouver dans l'art antérieur aucune description des éventuelles propriétés de ce composé, ni de sa méthode de préparation.

Par ailleurs, on trouve également dans la littérature des références aux 4-(2,2,3,6-tétraméthyl-1-cyclohexyl)-2-butanone et 4-(2,2,3,6-tétraméthyl-1-cyclohexyl)-3-butén-2-one. C'est ainsi que plusieurs formes isomériques de la première sont citées par A. Storni, thèse de doctorat, ETH, Juris-Verlag, Zürich (1962) dans le cadre d'une étude de la configuration de la $\gamma$-irone. D'autre part, la structure de la buténone susmentionnée est décrite par P. Bächli et al. dans Helv. Chim. Acta 34, 1168 (1951). Malgré leurs efforts, les auteurs de cette dernière référence n'ont pas réussi à identifier les différents isomères de l'énone citée. Aucun de ces travaux ne mentionne des propriétés odorantes particulières de ces cétones. En effet, ces études s'inséraient dans un programme plus vaste d'élucidation de la structure des irones, un sujet fort controversé à l'époque [voir, par exemple, Y.R Naves et al., Bull. Soc. Chim. France 1953, 112 et références y citées] et les propriétés odorantes particulières de leurs dérivés dihydro et tétrahydro susmentionnés sont passées complètement inaperçues.

Dans ce contexte, le fait que les propriétés olfactives de ces cétones n'aient pas éveillé la curiosité des parfumeurs à l'époque n'est pas surprenant, étant donné que leur structure est très proche de celle des irones, ionones et méthylionones, qui constituaient déjà une masse impressionnante de produits odorants bien connus et appréciés. Ils constituaient en effet le sujet primordial de recherche et rien n'aurait pu laisser présager que l'on trouverait encore de nouveaux composés odorants dans ce type de structure, dont les propriétés olfactives ne feraient pas double emploi avec celles des composés déjà connus.

Or, nous avons constaté que non seulement les cétones de formule (Ia) connues susmentionnées possédaient des propriétés odorantes intéressantes et différentes de celles des autres composés de structure semblable connus, mais que d'autres cétones nouvelles de structure analogue, faisant l'objet de la présente invention et obéissant à la formule générale (Ia) citée, pouvaient être avantageusement employées pour la préparation de compositions parfumantes et produits parfumés, en raison de l'originalité et individualité de leurs qualités olfactives.

Par ailleurs, nous avons également découvert que parmi les différents isomères possibles de cette série de composés (Ia), les isomères cyclaniques de configuration trans de formules (Ib) et (Ic) définies plus haut possédaient systématiquement des propriétés olfactives jugées supérieures à celles de leurs isomères cis correspondants. En particulier, nous avons pu établir que parmi les composés de formule (Ia) possédant un cycle saturé, les isomères dont les substituants en positions 1, 3 et 6 du cycle ont une configuration équatoriale, de formule (Ic) définie plus haut, possédaient des caractères odorants plus marqués et mieux appréciés que ceux des isomères correspondants de configuration différente.

Malgré leur structure très proche de celle des irones et ionones, et des légères variations structurelles d'un composé à l'autre, les composés de l'invention apportent au domaine des notes irisées-violette un riche éventail de tonalités olfactives, parfois assorties de variations remarquables dans l'intensité et ténacité de l'odeur.

Le Tableau suivant, qui résume les propriétés olfactives de composés préférés selon l'invention, éventuellement sous forme de plusieurs isomères, renforce ces observations.

TABLEAU

| N° | Composé | Propriétés olfactives |
|---|---|---|
| 1 | 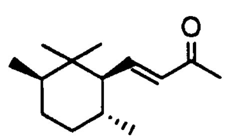 (racémique) | note iris-violette, poudrée, balsamique, rappelant la myrrhe, boisée ; intensité et ténacité remarquables |
| 2 | 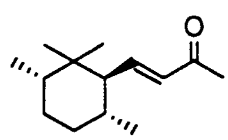 (racémique) | note boisée, similaire à celle de 1, moins ironée, beaucoup plus faible |
| 3 | 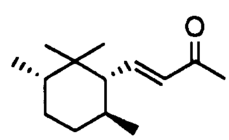 ($[\alpha]^{20}_D$=28,15°) | note irisée-ironée, avec un côté baumique-myrrhe et un côté boisé très chaud. Odeur un peu moins sèche-boisée que celle de 1, plus violette-iris et encore plus puissante |

TABLEAU (suite)

| N° | Composé | Propriétés olfactives |
|---|---|---|
| 4 | 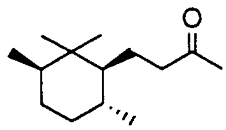 (racémique) | note plus faible et moins ironée que celle de 1 |
| 5 | 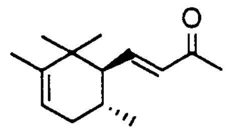 (racémique) | caractère boisé plus fort que 1 ; note très naturelle rappelant le cèdre et le santal, cuirée en fond |
| 6 | 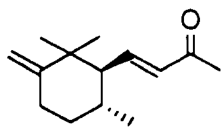 (racémique) | note iris-violette, joli côté boisé rappelant plutôt l'odeur des irones |
| 7 | 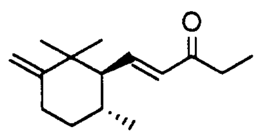 (racémique) | note fruitée-florale, abricot, freesia, boisée |

Cette variété olfactive entre composés de structure très proche est présente dans l'ensemble des autres composés (Ia) de l'invention dont les notes odorantes sont décrites dans les exemples de préparation présentés plus loin.

Parmi les composés de l'invention, et notamment ceux cités dans ce tableau, la (E)-4-(2,2,c-3,t-6-tétraméthyl-r-1-cyclohexyl)-3-butén-2-one (composé 1) est le composé qui possède les propriétés convenant le mieux à certaines applications en parfumerie selon l'invention. Son utilisation dans des compositions parfumantes, par exemple, confère à ces dernières une qualité poudrée et orientale qui se fait sentir dès les plus infimes concentrations du composé susmentionné, et qui étonne par sa richesse et sa performance lorsque comparée à celle des irones, ionones et méthylionones, et notamment de l'$\alpha$-irone. La ténacité de sa note s'est révélée bien supérieure à celle de ces produits connus, comme le montrent les résulats des tests comparatifs de substantivité sur linge présentés plus loin.

Cependant, les autres composés de l'invention trouvent également des applications avantageuses, selon le type d'effet olfactif recherché, la nature et le but du produit à parfumer.

Les composés de l'invention peuvent être employés dans des compositions parfumantes fort diverses, leur effet se faisant sentir de façon également heureuse dans des compositions de type féminin et masculin destinées à des parfums et eaux de toilette, auxquelles ils confèrent un caractère riche et chaud. Par ailleurs, ils sont également très appréciés pour le parfumage de savons et autres produits d'hygiène corporelle ou capillaire, tels que gels de douche ou bain, désodorisants, shampoings et crèmes pour les cheveux, ainsi que de préparations cosmétiques. Ils servent également au parfumage de produits d'entretien ou encore de détergents et revitalisants textiles, domaine dans lequel la (E)-4-(2,2,c-3,t-6-tétraméthyl-r-1-cyclohexyl)-3-butén-2-one se révèle encore être un produit de choix, de par la ténacité de son odeur.

Les proportions dans lesquelles les composés de formule (Ia-c) peuvent être utilisés pour obtenir les effets parfumants désirés peuvent varier dans une gamme de valeurs très étendue. L'homme du métier sait par expérience que de telles valeurs dépendent de l'effet particulier recherché, ainsi que de la nature des produits que l'on désire parfumer. On sait également que ces valeurs sont fonction de la nature des autres constituants dans une composition donnée, lorsque l'un des composés selon l'invention est utilisé en tant qu'ingrédient dans une base parfumante ou dans un concentré, en mélange avec d'autres coingrédients, des solvants ou des adjuvants usuels.

A titre d'exemple, on peut citer des proportions de l'ordre de 1 à 10%, voire 20% en poids par rapport au poids de la composition, lorsqu'il s'agit de la préparation de bases et concentrés parfumants. Ces

valeurs peuvent être bien inférieures lors du parfumage d'articles tels les savons et cosmétiques ou les détergents.

Selon l'invention, on utilisera l'un des composés de l'invention soit par adjonction directe à l'article que l'on désire parfumer, soit plus généralement en mélange avec d'autres ingrédients parfumants dont l'utilisation est courante en parfumerie. La mention spécifique de tels coingrédients est ici superflue. L'état de la technique en présente bien des exemples et l'homme du métier est à même de choisir ceux qui conviendront le mieux à l'effet odorant recherché. On citera comme exemple de référence l'ouvrage de S. Arctander, Perfume and Flavor Chemicals, Montclair, N.J., USA (1969).

Les compositions parfumantes et articles parfumés contenant, à titre d'ingrédient actif, un composé de formule (Ia) à (Ic) selon l'invention font également l'objet de cette dernière. On peut citer dans ce contexte les compositions parfumantes et articles parfumés qui résultent de l'utilisation des composés de l'invention cités à titre préférentiel et, en particulier, de l'utilisation de la (E)-4-(2,2,c-3,t-6-tétraméthyl-r-1-cyclohexyl)-3-butén-2-one, racémique ou optiquement active, à titre d'ingrédient parfumant.

L'invention sera maintenant décrite plus en détail à l'aide des exemples de préparation présentés ci-après dans lesquels les températures sont indiquées en degrés centigrades et les abréviations ont le sens usuel dans l'art.

L'invention sera également illustrée par des exemples d'application en parfumerie.

Exemple 1

Préparation d'époxydes de formule (II)

Méthode générale

La méthode utilisée pour la préparation de ces époxydes est illustrée par le schéma suivant :

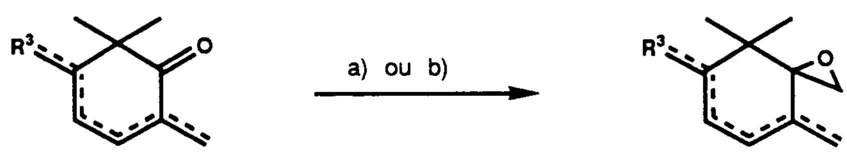


(V) (II)

a) $(CH_3)_3S^+CH_3SO_4^-$, NaH (ou NaOH), DMSO
b) $(CH_3)_3S^+X^-$ (X=Cl,I), NaH (NaOH), DMSO

Comme il est décrit dans la demande de brevet européen n° 0374509, le méthylure de diméthylsulfonium utilisé comme réactif dans la réaction de Corey-Chaykovsky a été formé in situ dans le milieu réactionnel par réaction soit de diméthylsulfure et diméthylsulfate, soit d'halogénure de triméthylsulfonium, avec l'hydrure ou l'hydroxyde de sodium dans le diméthylsulfoxyde (DMSO), et on a procédé comme suit.

Dans un réacteur à 3 cols muni d'un réfrigérant approprié, d'une ampoule d'introduction, d'une agitation mécanique et maintenu sous atmosphère inerte, on a introduit le sulfate de diméthyle et ajouté ensuite lentement le diméthylsulfure. La réaction exothermique a été tempérée à l'aide d'un bain de glace afin de maintenir un reflux du diméthylsulfure. Lorsque le reflux avait presque cessé, on a ajouté du DMSO pour dissoudre le sel formé. Le bain de refroidissement a été enlevé 10 minutes, pendant lesquelles on a continué le brassage, et ensuite on a refroidi de nouveau à 20°.

A cette température, on a ajouté l'hydrure de sodium à 75% dans de l'huile minérale (ou l'hydroxyde de sodium en perles). Après agitation pendant environ 30 min, on a ajouté, à une température pouvant varier, selon les cas, entre 0° et 6°, la cétone appropriée en solution dans le DMSO. Le mélange réactionel a ensuite été maintenu sous agitation, à température ambiante, pendant une période suffisante pour que la disparition de la cétone de départ soit complète.

On a ensuite ajouté de l'acétate d'éthyle, suivi lentement de l'addition d'eau. La phase aqueuse a été extraite successivement à l'éther de pétrole (30-50°), acétate d'éthyle et, de nouveau, éther de pétrole (30-50°). Les phases organiques ont été réunies et lavées à neutralité avec une solution appropriée et ensuite avec de l'eau. On a séché sur $Na_2SO_4$ ou $MgSO_4$, filtré et évaporé pour obtenir un produit brut. La

distillation au four à boules de ce dernier a permis d'obtenir le produit final désiré à l'état pur.

Lorsqu'on a utilisé de l'halogénure de triméthylsulfonium comme réactif, au lieu de diméthylsulfate et diméthylsulfure, l'hydrure de sodium (ou le NaOH) a été ajouté à une solution dudit réactif dans le DMSO, dans un réacteur du type décrit précédemment, et la cétone appropriée a été ajoutée en solution dans le DMSO. La procédure a été poursuivie comme décrit auparavant.

Selon cette méthode générale, on a préparé les époxydes suivants.

**a**. trans-4,4,8-triméthyl-1-oxaspiro[2.5]octane et cis-4,4,8-triméthyl-1-oxaspiro[2.5]octane

Préparés dans un réacteur à 3 cols muni d'un réfrigérant à glace sèche et sous argon. On a utilisé 30 g (239 mmole) de $(CH_3)_2SO_4$, 16 g (257 mmole) de $(CH_3)_2S$, 50 ml de DMSO et 41 g (1026 mmole) de NaOH. Une solution de 2,2,6-triméthylcyclohexanone (24 g, 171 mmole) dans 25 ml de DMSO a été ajoutée à 6°. Le mélange a été brassé pendant 6 h. On a ajouté 100 ml d'acétate d'éthyle, puis, à 0°, 300 ml d'eau. L'extraction a été effectuée avec 2x100 ml d'éther de pétrole et 100 ml d'acétate d'éthyle. On a lavé avec 2x100 ml de solution saturée en NaCl et 100 ml d'eau. Après séchage et filtration, 23,6 g d'huile brute ont été distillés pour fournir 21,4 g d'un mélange pur à 94%, contenant 68% de l'isomère trans et 32% de l'isomère cis susmentionnés.

P. éb.: 100°/6x$10^2$ Pa ; rend. 76%

IR : 2980, 1480, 1395 $cm^{-1}$

isomère cis

RMN($^1$H,360MHz) : 0,70(d,J = 7Hz,3H) ; 0,75(s,3H) ; 1,08(s,3H) ; 1,20-2,20 (m,7H) ; 2,63(s,2H) δ ppm

RMN($^{13}$C) : 14,9(q) ; 22,2(t) ; 24,4(q) ; 24,5(q) ; 30,2(d) ; 33,2(t) ; 34,4(s) ; 38,3(t) ; 45,9(t) ; 64,3(s) δ ppm

SM : 154(M$^+$,9), 139(45), 109(100), 81(57), 69(52), 67(78), 55(53), 41(69)

isomère trans

RMN($^1$H,360MHz) : 0,72(d,J = 7Hz,3H) ; 0,77(s,3H) ; 1,07(s,3H) ; 1,20-2,20(m,7H) ; 2,65-(AB,J = 5Hz,Δν = 32Hz,2H) δ ppm

RMN($^{13}$C) : 15,0(q) ; 21,7(t) ; 22,5(q) ; 25,7(q) ; 31,6(d) ; 34,7(s) ; 35,2(t) ; 40,5(t) ; 45,9(t) ; 65,4(s) δ ppm

SM : 154(M$^+$,9), 139(42), 109(100), 81(52), 69(58), 67(76), 55(55), 41(69)

**b**. 4,8,8-triméthyl-1-oxaspiro[2.5]oct-4-ène

Préparé dans les conditions décrites sous a., à partir de 25,0 g (181 mmole) de 2,6,6-triméthyl-2-cyclohexén-1-one, 31,9 g (253 mmole) de $(CH_3)_2SO_4$, 17,0 g (274 mmole) de $(CH_3)_2S$ et 43,0 g (1086 mmole) de NaOH dans 60 ml de DMSO. On a obtenu 22,09 g du produit désiré pur à 91%.

P. éb. : 100°/6x$10^2$ Pa; rend. 73%

IR : 2970, 1680, 1450 $cm^{-1}$

RMN($^1$H,360MHz) : 0,88(s,3H) ; 0,92(s,3H) ; 1,53(s,3H) ; 2,87(AB,J = 4Hz, Δν = 15Hz,2H) ; 5,77(s large,1H) δ ppm

RMN($^{13}$C) : 17,1(q) ; 22,6(q) ; 22,8(t) ; 24,3(q) ; 32,15(s) ; 35,5(t) ; 48,1(t) ; 62,7(s) ; 129,2(d) ; 129,2(s) δ ppm

SM : 152(M$^+$,12), 137(42), 124(100), 107(77), 91(55), 79(55), 41(42)

**c**. 4,4-diméthyl-8-méthylène-1-oxaspiro[2.5]octane

Préparé selon la méthode générale décrite, à partir de 4,5 g (25,0 mmole) de 2,2-diméthyl-6-méthylène-1-cyclohexanone pure à 75% dans 5 ml de DMSO, ajoutés à une solution de 4,8 g (42,4 mmole) de chlorure de triméthylsulfonium et 13,0 g (326 mmole) d'hydroxyde de sodium dans 30 ml de DMSO. Le mélange a été brassé pendant 3 h et on a ajouté 50 ml d'acétate d'éthyle, puis 100 ml d'eau. La phase aqueuse a été extraite avec 3x50 ml d'acétate d'éthyle et la phase organique a été lavée avec une solution à 10% de HCl et de l'eau et séchée sur $MgSO_4$. La distillation a fourni 1,25 g de l'époxyde désiré (94% pur, rend. 32%).

IR : 3060, 2950, 1660, 1460 $cm^{-1}$

RMN($^1$H,360MHz) : 0,83(s,3H) ; 0,94(s,3H) ; 2,45(d,J = 5Hz,1H) ; 2,93(d, J = 5Hz,1H) ; 4,75(s large,1H) ; 4,90(s large,1H) δ ppm

RMN($^{13}$C) : 22,65(t) ; 22,75(q) ; 24,3(q) ; 34,79(s) ; 34,92(t) ; 39,6(t) ; 52,5(t) ; 65,1(s) ; 107,49(t) ; 146,76(s) δ ppm

SM : 152(M$^+$,8), 137(100), 123(33), 109(89), 96(45), 91(50), 81(89), 79(70), 69(63), 67(66), 41(80).

**d.** 4,4,t-5,r-8-tétraméthyl-1-oxaspiro[2.5]octane

Préparé selon la méthode générale décrite, à partir de la 2,2,3,6-tétraméthyl-1-cyclohexanone (mélange trans/cis : 85/15%) dans les conditions énoncées dans la demande de brevet européen n° 0374509. Les

données analytiques de cet époxyde y sont également décrites. Lorsqu'on a utilisé comme produit de départ la (+)-(3S,6S)-2,2,3,6-tétraméthyl-1-cyclohexanone ($[\alpha]^{20}D$ = +52,2°), ou respectivement la (-)-(3R,6R)-2,2,3,6-tétraméthyl-1-cyclohexanone ($[a]^{20}D$ = -50,7°), on a obtenu des isomères optiquement actifs de l'époxyde susmentionné, à savoir (+)-(3S,5S,8S)-4,4,5,8-tétraméthyl-1-oxaspiro[2.5]octane ($[\alpha]^{20}D$ = +36,0°), ou respectivement (-)-(3R,5R,8R)-4,4,5,8-tétraméthyl-1-oxaspiro [2.5]octane ($[\alpha]^{20}D$ = -35,1°). Les données analytiques de ces composés sont aussi décrites dans la demande de brevet européen susmentionnée.

e. (3RS,7SR)-4,7,8,8-tétraméthyl-1-oxaspiro[2.5]oct-4-ène

Préparé selon la méthode générale décrite, à partir de 40 g (263 mmole) de 2,5,6,6-tétraméthyl-2-cyclohexén-1-one et 1,6 équivalent de $(CH_3)_2S/(CH_3)_2SO_4$/NaH dans 40 ml de DMSO. Après 15 h de réaction, on a ajouté 100 ml d'acétate d'éthyle et ensuite, goutte à goutte, 100 ml d'eau. La phase aqueuse a été lavée avec 100 ml d'éther de pétrole (30-50°) et 100 ml d'acétate d'éthyle. Les phases organiques ont été réunies et lavées avec 2x100 ml d'eau, séchées sur $Na_2SO_4$ et évaporées. Après distillation du produit brut (42,3 g), constitué par un mélange contenant, outre le composé désiré, 9% de la cétone de départ, on a obtenu l'époxyde susmentionné.

| | |
|---|---|
| IR : | 3020, 2900, 1440, 1360 $cm^{-1}$ |
| RMN($^1$H,360MHz) : | 0,78(s,3H) ; 0,86(s,3H) ; 0,91(d,J=7Hz,3H) ; 1,50 (s large,3H) ; 2,77-(AB,J=4Hz,$\Delta\nu$=50Hz,2H) ; 5,66(s large,1H) $\delta$ ppm |
| SM : | 166($M^+$,10), 151(35), 137(30), 124(100), 121(50), 109(32), 105(40), 95(40), 91-(35), 79(35), 41(55). |

En utilisant comme produit de départ la (+)-(5S)-2,5,6,6-tétraméthyl-2-cyclohexén-1-one ($[\alpha]^{20}D$ = +79,5°), on a obtenu suivant la même méthode le (+)-(3R,7S)-4,7,8,8-tétraméthyl-1-oxaspiro[2.5]-oct-4-ène ($[\alpha]^{20}D$ = +53°).

**f.** (+)-(3R,5S)-4,4,5-triméthyl-8-méthylèn-1-oxaspiro[2.5]octane

Préparé à partir de 10,4 g (0,092 mole) de chlorure de triméthyl-sulfonium, 10 ml de DMSO et 13,15 g (0,33 mole) de NaOH en pastilles (ajoutés d'un coup). Après refroidissement avec un bain de glace, on a introduit goutte à goutte et sous vive agitation 10 g (0,066 mole) de (+)-(3S)-2,2,3-triméthyl-6-méthylène-1-cyclohexanone ($[\alpha]^{20}D$ = +6,7°, 5% $CHCl_3$) en solution dans 10 ml de DMSO, sans laisser la température dépasser 6°. Après une nuit de brassage à température ambiante, on a refroidi avec un bain de glace et hydrolysé, sans dépasser 20°, avec de l'eau. Après le traitement habituel, on a obtenu 8,6 g de produit brut. Ce dernier a été distillé sur résidus et ensuite, finement, sur colonne Fischer. On a obtenu 1,6 g d'époxyde pur à 75%.

$[\alpha]^{20}D$ = +22,8°, c=5,2% $CHCl_3$

| | |
|---|---|
| P. éb. : | 58°/0,9x$10^2$ Pa; rend. 15% |
| IR : | 3060, 2990, 2950, 1660, 1460 $cm^{-1}$ |
| RMN($^1$H,360MHz) : | 0,77(s,3H) ; 0,83(s,3H) ; 0,90(d,J=7Hz,3H) ; 2,33(d, J=7Hz,1H) ; 2,90-(d,J=7Hz,1H) ; 4,69(s large,1H) ; 4,92(s large,1H) $\delta$ ppm |
| RMN($^{13}$C) : | 15,6(q) ; 16,7(q) ; 21,35(q) ; 31,1(t) ; 34,0(t) ; 37,85(s) ; 41,36(d) ; 52,86(t) ; 65,32(s) ; 106,4(t) ; 145,56(s) $\delta$ ppm |
| SM : | 166($M^+$,9), 151(50), 137(21), 123(33), 109(60), 95(100), 81(68), 67(38), 55(40), 41(17) |

**g**. (+)-(3R,7S)-7-éthyl-4,8,8-triméthyl-1-oxaspiro[2.5]oct-4-ène

Préparé selon la méthode décrite, dans un réacteur muni d'un réfrigérant à $CO_2$ et maintenu sous $N_2$, à partir de 6,8 g (0,054 mole) de $(CH_3)_2SO_4$, 3,3 g de $(CH_3)_2S$, 6 ml de THF (tétrahydrofuranne), 8 ml de DMSO et 1,7 g (0,054 mole) de NaH à 75% dans de l'huile minérale. La (+)-(5S)-5-éthyl-2,6,6-triméthyl-2-cyclohexén-1-one (6,6 g, 0,033 mole, $[\alpha]^{20}D$ = +82,4°), dans 2 ml de DMSO, a été ajoutée à ≈6°. On a brassé pendant un week-end, ajouté 21 ml d'acétate d'éthyle, puis goutte à goutte 21 ml d'eau. Après le traitement habituel et distillation sur résidus, on a obtenu un mélange contenant 54% du produit désiré. Ce dernier a été séparé par une nouvelle distillation.

$[\alpha]^{20}D$ = +54°

| | |
|---|---|
| IR : | 2975, 1670, 1460, 1380 $cm^{-1}$ |
| RMN($^1$H,360MHz) : | 0,80(s,3H) ; 0,87(s,3H) ; 0,89(t,J≈7Hz,3H) ; 1,51(d, J≈2Hz,3H) ; 2,77-(AB,J=5Hz,$\Delta\nu$=51Hz,2H) ; 5,69(s large,1H) $\delta$ ppm |
| SM : | 180($M^+$,5), 165(23), 151(30), 137(22), 133(10), 124(100), 115(2), 105(31), 91-(23), 84(7), 79(18), 73(5), 69(15), 59(8), 55(18), 41(55) |

Exemple 2

Préparation d'esters de formule (VI)

**a**. 2,2,6-triméthyl-3-méthylèn-1-cyclohexanecarboxylate de méthyle

Dans un ballon à 3 cols muni d'un réfrigérant, d'un thermomètre et d'une ampoule d'introduction, maintenu sous $N_2$, on a placé 156 g (0,614 mole) de 6-(acétoxyméthyl)-3,7-diméthyl-2,7-octadiénoate de méthyle (mélange trans/cis) [obtenu à partir de géraniate de méthyle par une réaction du type de Prins-Blomquist ; voir A.T. Blomquist et al., J. Org. Chem. 33, 1156 (1968)] et 750 ml d'acide acétique et on a introduit assez rapidement 30 ml de $SnCl_4$. La réaction était légèrement exothermique. On a laissé brasser pendant environ 15 h à température ambiante. Le mélange de la réaction a été versé sur NaCl saturé, extrait à l'éther et lavé 5 fois avec une solution de NaCl et une fois avec une solution de $NaHCO_3$. On a séché sur $Na_2SO_4$, filtré, concentré au rotavapeur et distillé sur résidus. On a obtenu 84 g d'un mélange d'isomères non déterminés du 2-(acétoxyméthyl)-2,6,6-triméthyl-1-cyclohexène-1-carboxylate de méthyle. Ce mélange a été utilisé tel quel dans la poursuite de la synthèse.

P. éb. : 95-105 °/ 6 Pa ; rend. 54%

70 g (0,28 mole) du mélange susmentionné, en solution dans 500 ml d'acide acétique ont été hydrogénés à pression atmosphérique et température ambiante en présence de 0,3 g de $PtO_2$. Après une nuit, on a constaté une consommation d'environ 7 l de $H_2$ (0,28 mole). On a filtré, concentré et distillé sur résidus le produit de la réaction pour obtenir 67,7g d'un mélange d'isomères non déterminés du 3-(acétoxyméthyl)-2,2,6-triméthyl-1-cyclohexanecarboxylate de méthyle. Ce mélange a été utilisé tel quel dans la poursuite de la synthèse.

P. éb. : 79-88° / 6 Pa ; rend. 96%

66 g (0,25 mole) du mélange d'isomères décrit en dernier lieu ont été passés en 30 h dans une colonne en pyrex de 5 m, chauffée à 430° et entraînés par un faible courant d'azote. Le pyrolisat a ensuite été distillé sur résidus pour fournir 46,1 g d'une huile brune (p. éb. : 58-66°/5x10 Pa, 65% pur). Le fractionnement de ce produit brut a fourni 28,3 g de 2,2,6-triméthyl-3-méthylène-1-cyclohexan-carboxylate de méthyle (2 isomères, cis/trans ≈ 9:1) sous forme d'huile incolore.

| | |
|---|---|
| P. éb. : | 42-43°/16x$10^2$ Pa |
| IR : | 3030, 2950, 1740, 1640, 1440, 1130, 890 $cm^{-1}$ |
| RMN($^1$H,360MHz) : | 0,89(d,J=7,2Hz,3H) ; 1,09(s,3H) ; 1,17(s,3H) ; 1,53(m,1H) ; 1,74(m,1H) ; 2,14-(m,1H) ; 2,23-2,43(m,3H) ; 3,60(s,3H) ; 4,66(s,1H) ; 4,78(s,1H) δ ppm |
| SM : | 196($M^+$,1), 164(18), 149(3), 136(23), 121(88), 107(28), 93(35), 81(42), 67(37), 59(26), 55(51), 41(100) |

**b.** 2,2,3,6-tétraméthyl-3-cyclohexène-1-carboxylate de méthyle

13 g (0,066 mole) du 2,2,6-triméthyl-3-méthylène-1-cyclohexane carboxylate de méthyle obtenu sous a., en solution dans 130 ml de toluène et en présence de 0,5 g d'acide paratoluène-sulfonique, ont été chauffés à reflux pendant 2 h sous azote. On a lavé le mélange de la réaction avec successivement une solution de $NaHCO_3$ et de NaCl jusqu'à neutralité, séché sur $Na_2SO_4$, concentré et distillé sur résidus. On a obtenu 11,7 g de l'ester désiré (2 isomères, cis/trans ≈ 9:1) sous forme d'une huile incolore.

| | |
|---|---|
| P. éb. : | 40-43°/1x10 Pa ; rend. 90% |
| IR : | 2950, 1740, 1440, 1120 $cm^{-1}$ |
| RMN($^1$H,360MHz) : | 0,94(d,J=7,2Hz,3H) ; 1,02(s,3H) ; 1,14(s,3H) ; 1,66(s large, 3H) ; 1,90-2,20-(m,3H) ; 2,42(d,J=3,6Hz,1H) ; 3,63(s,3H) ; 5,4(s large,1H) δ ppm |
| SM : | 196($M^+$,6), 164(27), 149(4), 136(23), 121(100), 107(13), 96(18), 81(19), 67(10), 41(10) |
| Odeur : | fruitée, safran. |

Exemple 3

Préparation d'alcools de formule (VII)

Ces composés ont été préparés à partir des esters décrits dans l'Exemple 2.

**a**. 2,2,6-triméthyl-3-méthylène-1-cyclohexaneméthanol

Dans un ballon à 3 cols, muni d'un thermomètre, d'un réfrigérant et d'une ampoule d'introduction, maintenu sous $N_2$, on a placé 1,5 g (0,04 mole) de $LiAlH_4$ en suspension dans 40 ml d'éther sulfurique (sec) et introduit goutte à goutte une solution de 8,0 g (0,04 mole) de l'ester préparé selon l'Exemple 2a. et de 40 ml d'éther sulfurique (sec), tout en faisant réagir au reflux. On a brassé encore pendant 1 h à

température ambiante et ensuite refroidi à 0° (bain glacé). A cette température, on a introduit avec précaution et successivement 1,5 ml de $H_2O$, 1,5 ml de NaOH (15%) et 4,5 ml de $H_2O$. Après 1/2 h de brassage, on a filtré sur verre fritté, bien rincé à l'éther, concentré et distillé au four à boules. On a obtenu 6,45 g de l'alcool désiré (2 isomères, cis/trans ≈ 9:1) sous forme d'une huile incolore.

P. éb. : 150°/3x$10^2$ Pa ; rend. 94%
IR : 3400, 3120, 2950, 1660, 1460, 1010, 900 $cm^{-1}$
RMN($^1$H,360MHz,$D_2O$) : 1,03(d,J = 7,2Hz,3H) ; 1,18(s,3H) ; 1,25(s,3H) ; 1,25(m,1H) ; 1,31(m,1H) ; 1,60(m,1H) ; 2,21(m,2H) ; 2,40(m,1H) ; 3,59(dd,$J_1$ = 3,6,$J_2$ = 10,8Hz,1H) ; 3,76(dd,$J_1$ = 2,5Hz,$J_2$ = 10,8Hz,1H) ; 4,75(s large,1H) ; 4,76(s large,1H) δ ppm
RMN($^{13}$C) : 155,9(s) ; 107,3(t) ; 60,9(t) ; 54,4(d) ; 39,7(s) ; 32,9(t) ; 32,6(t) ; 30,1(d) ; 28,5(q) ; 26,6(q) ; 19,7(q) δ ppm
SM : 168($M^+$,3), 153(9), 135(80), 121(28), 107(100), 95(75), 84(96), 67(68), 55-(49), 41(23)

**b**. 2,2,3,6-tétraméthyl-3-cyclohexène-1-méthanol

Préparé selon la méthode décrite sous a. avec 1,9 g (0,05 mole) de $LiAlH_4$ en suspension dans 60 ml d'éther sulfurique, 10,0 g (0,05 mole) de l'ester préparé selon l'Exemple 2b. et 40 ml d'éther. Le traitement du mélange de la réaction a été effectué avec, successivement, 1,9 ml d'eau, 1,9 ml de NaOH et 5,7 ml d'eau. On a obtenu 8,4 g d'un mélange contenant 90% de l'isomère cis et 10% de l'isomère trans du méthanol susmentionné (huile incolore).

P. éb. : 45°/5 Pa; rend. 97%

L'isomère cis majoritaire désiré a été purifié par chromatographie en phase gazeuse. Ses données analytiques étaient les suivantes :

IR : 3360, 3000, 1460, 1060, 820 $cm^{-1}$
RMN($^1$H,360MHz) : 1,05(d,J = 7,2Hz,3H) ; 1,14(s,6H) ; 1,34(m,1H) ; 1,66(s large, 3H) ; 1,72(m,1H) ; 1,98(m,1H) ; 2,0(m,1H) ; 3,73(m,2H) ; 5,32(m,1H) δ ppm
SM : 168($M^+$,12), 153(9), 146(3), 135(89), 119(41), 107(100), 95(68), 91(48), 84(40), 81(65), 67(33), 55(27), 43(20)

Exemple 4

Préparation d'aldéhydes de formule (III) à partir d'époxydes (II) Méthode générale

Cette méthode peut être illustrée par le schéma suivant :

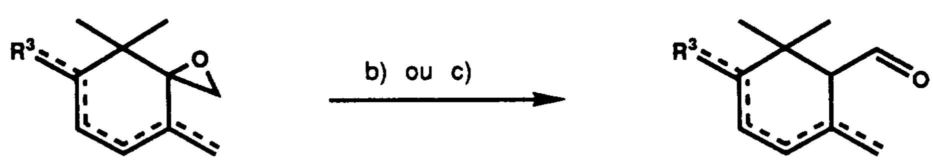


(II) (III)

b) $MgI_2$, toluène
c) $SnCl_4$ cat./toluène, 0°

La transformation de l'époxyde (II) en l'aldéhyde (III) à l'aide de $MgI_2$ dans le toluène permet, en fait, la synthèse en un seul pot des cétones cycliques de formule (I) possédant un cycle saturé, sans qu'il y ait besoin de séparer l'aldéhyde (III) du mélange réactionnel. Le $MgI_2$ a été préparé in situ, en atmosphère inerte à partir de magnésium et iode dans le tétrahydofuranne (THF). Ce dernier a ensuite été évaporé sous pression réduite et on a ajouté du toluène sec que l'on a porté à reflux pour dissoudre l'iodure de magnésium. Après une nuit au repos à 25°, on a ajouté l'époxyde approprié après avoir porté le mélange à reflux. On a laissé refluer sous agitation pendant 30-40 min. L'aldéhyde ainsi formé a ensuite été soit séparé après épimérisation du mélange à l'aide de $NaOCH_3$ dans le méthanol, soit directement utilisé dans la préparation de la cétone correspondante, comme il est décrit dans l'Exemple 6a.

Lorsque l'époxyde (II) a été transformé en l'aldéhyde (III) à l'aide de $SnCl_4$ dans le toluène, on a procédé

comme suit.

Dans un réacteur sous azote, maintenu à 0°, on a ajouté le $SnCl_4$ à une solution de l'époxyde approprié dans le toluène. Après quelques minutes de brassage, on a neutralisé le mélange de la réaction avec une solution adéquate et ensuite ajouté de l'eau. La phase aqueuse a été extraite avec de l'éther de pétrole (30-50°) et la phase organique a été lavée à l'eau. On a séché sur $Na_2SO_4$ et concentré. Le produit brut a été purifié à l'aide des techniques usuelles.

Par ailleurs, des aldéhydes de formule (IIIb) ont été préparés par isomérisation de leurs isomères de configuration α, dans les conditions décrites plus loin cas par cas.

On a préparé les aldéhydes suivants.

**a**. trans-2,2,6-triméthyl-1-cyclohexanecarbaldéhyde et cis-2,2,6-triméthyl-1-cyclohexanecarbaldéhyde

On a fait réagir 20 g (129,9 mmole) de l'époxyde préparé selon l'Exemple 1a. (mélange cis/trans ≈ 68:32) avec l'iodure de magnésium [préparé à partir de 517 mg (21,5 mmole) de Mg et 5,09 g (20 mmole) de $I_2$ dans 20 ml de THF] dissous dans 30 ml de toluène. Après distillation au four à boules (100°/6x$10^2$ Pa), on a obtenu 18,74g (rend. 86%) d'un mélange pur à 92% contenant les deux isomères de l'aldéhyde désiré dans un rapport cis/trans = 43:57. Ce mélange a été enrichi en isomère trans par épimérisation dans 40 ml de méthanol, à l'aide de 3 g d'une solution à 30% de $NaOCH_3$ dans le méthanol. Après 18 h de réaction, on a obtenu 15,6 g (rend. 74%) d'un mélange pur à 95% contenant 95% de trans-2,2,6-triméthyl-1-cyclohexanecarbaldéhyde et 5% de cis-2,2,6-triméthyl-1-cyclohexanecarbaldéhyde.

trans-2,2,6-triméthyl-1-cyclohexanecarbaldéhyde

| | |
|---|---|
| IR : | 2960, 1715, 1455 $cm^{-1}$ |
| RMN($^1$H,360MHz) : | 0,81(d,J=7Hz,3H) ; 0,97(s,3H) ; 1,01(s,3H) ; 1,10-2,00(m,8H) ; 9,63-(d,J=6Hz,1H) δ ppm |
| SM : | 154($M^+$,23), 139(20), 121(32), 111(26), 83(47), 69(100), 55(54), 41(57) |

çis-2,2,6-triméthyl-1-cyclohexanecarbaldéhyde

| | |
|---|---|
| IR : | 2970, 1715, 1440 $cm^{-1}$ |
| RMN($^1$H,360MHz) : | 0,92(d,J=7Hz,3H) ; 0,93(s,3H) ; 1,03(s,3H) ; 1,30-2,05(m,8H) ; 9,60-(d,J=7Hz,1H) δ ppm |
| SM : | 154($M^+$,17), 139(16), 121(26), 111(24), 95(30), 85(63), 69(100), 55(68), 41(80) |

**b**. 2,2,c-3,t-6-tétraméthyl-r-1-cyclohexanecarbaldéhyde et 2,2,t-3,c-6-tétraméthyl-r-1-cyclohexanecarbaldéhyde

La préparation de ces deux aldéhydes, à partir de l'époxyde obtenu selon l'Exemple 1d., a été décrite dans la demande de brevet européen n° 0374509. Les données analytiques respectives y sont également décrites. Odeur : boisée, épicée, patchouli.

Un énantiomère optiquement actif du 2,2,c-3,t-6-tétraméthyl-r-1-cyclohexanecarbaldéhyde, à savoir le (+)-(1R,3S,6S)-2,2,3,6-tétraméthyl-1-cyclohexanecarbaldéhyde [contenant 10% de son diastéréoisomère (1S,3S,6S)] a été préparé de façon analogue, à partir du (+)-(3S,5S,8S)-4,4,5,8-tétraméthyl-1-oxaspiro-[2.5]octane décrit dans l'exemple 1d. L'aldéhyde optiquement actif susmentionné était caractérisé par $[\alpha]^{20}D$ = +7,4°, c=2,5% $CHCl_3$.

**c**. 2,6,6-triméthyl-2-cyclohexène-1-carbaldéhyde

Préparé par réaction de 0,1 ml (0,85 mmole) de $SnCl_4$ avec une solution de 6,6 g (43,42 mmole, 95% pur) de l'époxyde obtenu selon l'Exemple 1b. dans 66 ml de toluène. Après 20 min d'agitation, on a ajouté quelques gouttes d'une solution saturée de $NaHCO_3$, puis 20 ml d'eau. On a extrait la phase aqueuse avec 3x20 ml de toluène et lavé la phase organique avec 2x30 ml d'eau. Après séchage sur $MgSO_4$, évaporation et distillation au four à boules, on a obtenu 4,88 g (94% pur) du carbaldéhyde susmentionné, ou α-cyclocitral.

| | |
|---|---|
| P. éb. : | 100°/6x$10^2$ Pa; rend. 73% |
| IR : | 2980, 1720, 1680, 1450 $cm^{-1}$ |
| RMN($^1$H,60MHz) : | 0,87(s,3H) ; 0,96(s,3H) ; 1,62(s large,3H) ; 5,75(m,1H) ; 9,50(d,J=5Hz,1H) δ ppm |
| SM : | 152($M^+$,5), 123(56), 94(45), 81(100), 67(30) |

**d**. 2,6,6-triméthyl-1-cyclohexène-1-carbaldéhyde

Cet isomère β de l'aldéhyde préparé selon c. a été obtenu par isomérisation de 4,4 g (28,95 mmole) de ce dernier à l'aide de 1 ml de solution à 30% de $NaOCH_3$ dans le méthanol (≈ 5,6 mmole), à 20°, durant 3 h. On a évaporé le méthanol et remplacé par l'acétate d'éthyle. La phase organique a été lavée avec 10 ml d'une solution aqueuse à 10% de HCl puis 2x20 ml d'eau. On a séché sur $MgSO_4$, évaporé et distillé l'huile ainsi obtenue au four à boules. 4,25 g du carbaldéhyde susmentionné, ou β-cyclocitral, ont été obtenus (94% pur, rend. 73%).

| | |
|---|---|
| IR : | 2980, 1720, 1675, 1615, 1455 $cm^{-1}$ |

RMN($^1$H,60MHz) : 1,20(s,6H) ; 2,10(s,3H) ; 10,12(s,1H) δ ppm

SM : 152($M^+$,80), 137(100), 123(85), 109(92), 95(46), 81(82), 67(85)

**e.** 2,2-diméthyl-6-méthylène-1-cyclohexanecarbaldéhyde

Obtenu par réaction de 1,1 g (6,8 mmole, 94% pur) de l'époxyde préparé selon l'Exemple 1c., dans 10 ml de toluène, avec 40 µl (0,34 mmole) de $SnCl_4$, en 10 min. Après le traitement habituel, on a obtenu 0,84 g de produit brut, dont 96,5% ont été convertis en le carbaldéhyde désiré pur, ou γ-cyclocitral, par distillation au four à boules.

P. éb.: 100°/6x10$^2$ Pa; rend. 78%

IR : 3100, 2960, 2740, 1725, 1650, 1460 $cm^{-1}$

RMN($^1$H,360MHz) : 0,95(s,3H) ; 1,07(s,3H) ; 2,20(m,2H) ; 2,68(d,J=4Hz,1H) ; 4,70(s,1H) ; 4,95-(s,1H) ; 9,84(d,J=4Hz,1H) δ ppm

RMN($^{13}$C) : 22,85(t) ; 26,0(q) ; 28,15(q) ; 33,4(t) ; 34,95(s) ; 37,58(t) ; 66,64(d) ; 112,4(t) ; 143,6(s) ; 203,08(d) δ ppm

SM : 152($M^+$,7), 137(24), 123(50), 109(90), 95(43), 94(43), 81(100), 69(71), 41(80)

**f.** trans-2,5,6,6-tétraméthyl-2-cyclohexène-1-carbaldéhyde et cis-2,5,6,6-tétraméthyl-2-cyclohexène-1-carbaldéhyde

On a fait réagir 2,7 ml (23 mmole) de $SnCl_4$ avec une solution de 43 g (243,5 mmole) de l'époxyde obtenu selon l'Exemple 1e. (94% pur) dans 200 ml de toluène. Après 5 min, on a neutralisé avec une solution saturée de $NaHCO_3$, puis on a ajouté 100 ml d'eau. La phase aqueuse a été extraite avec 2x100 ml d'éther de pétrole (30-50°) et la phase organique lavée à l'aide de 2,50 ml d'eau. Après séchage sur $Na_2SO_4$ et évaporation, on a obtenu un produit brut sous forme d'une huile incolore (41,7 g). Ce produit a été distillé à l'aide d'une colonne Vigreux de 12 cm pour fournir un mélange contenant 48% de l'isomère trans, 43% de l'isomère β des carbaldéhydes susmentionnés, ou 2,5,6,6-tétraméthyl-1-cyclohexène-1-carbaldéhyde.

P. éb. : 70-85°/4x10$^2$ Pa; rend. 77%

Les deux composés isomères désirés ont été séparés du mélange susmentionné par chromatographie préparative en phase gazeuse et présentaient les données analytiques suivantes :

trans-2,5,6,6-tétraméthyl-2-cyclohexène-1-carbaldéhyde

IR : 2900, 1718, 1455, 1380 $cm^{-1}$

RMN($^1$H,360MHz) : 0,80(s,3H) ; 0,89(d,J=7Hz,3H) ; 1,00(s,3H) ; 1,59(s large, 3H) ; 2,36-(d,J=6Hz,1H) ; 5,70(s large,1H) ; 9,57(d,J=6Hz,1H) δ ppm

SM : 166($M^+$,15), 137(62), 108(52), 95(100), 81(62), 67(37), 55(50), 41(85)

cis-2,5,6,6-tétraméthyl-2-cyclohexène-1-carbaldéhyde

IR : 2900, 1718, 1455, 1380 $cm^{-1}$

RMN($^1$H,360MHz) : 0,87(d,J=7Hz,3H) ; 0,90(s,3H) ; 0,99(s,3H) ; 1,63(s large, 3H) ; 2,57(s large,1H) ; 5,66(s large,1H) ; 9,64(d,J=5Hz,1H) δ ppm

SM : 166($M^+$,11), 137(58), 108(52), 95(100), 81(58), 55(55), 41(82)

**g.** 2,5,6,6-tétraméthyl-1-cyclohexène-1-carbaldéhyde

Préparé à partir du mélange d'aldéhydes décrit en f. (7g, 42,17 mmole) dans 50 ml de méthanol, par réaction avec 1 ml de $NaOCH_3$ à 30% dans le méthanol (≈ 5,5 mmole). Après 20 min, on a ajouté 100 ml d'éther de pétrole (30-50°), puis neutralisé avec une solution aqueuse à 10% de HCl. On a ajouté 20 ml d'une solution aqueuse saturée de NaCl et ensuite extrait avec 4x50 ml d'éther de pétrole. On a lavé la phase organique (2x50 ml NaCl saturé), repris les phases aqueuses avec 50 ml d'éther de pétrole, séché sur $Na_2SO_4$, évaporé et distillé au four à boules (P. éb. ≈ 90°/6x10$^2$ Pa).

On a obtenu 6,15g de produit désiré à 97% pur (rend. 88%).

IR : 2960, 1660, 1375, 1280 $cm^{-1}$

RMN($^1$H,360MHz) : 0,91(d,J=7Hz,3H) ; 1,06(s,3H) ; 1,22(s,3H) ; 2,09(s,3H) ; 10,12(s,1H) δ ppm

SM : 166($M^+$,70), 151(63), 148(35), 133(40), 123(48), 109(77), 95(53), 81(100), 41-(91)

Odeur : camphre, épicée, menthée, damasconée.

**h.** (-)-(1R,3S)-2,2,3-triméthyl-6-méthylène-1-cyclohexanecarbaldéhyde et (1S,3S)-2,2,3-triméthyl-6-méthylène-1-cyclohexanecarbaldéhyde

Préparés à partir de l'époxyde (+) décrit dans l'Exemple 1f. (0,85 g, 5 mmole), par réaction avec 0,06 ml (0,5 mmole) de $SnCl_4$, dans le toluène (10 ml). Après 10 min, on a repris dans un décanteur, lavé 2x avec HCl à 15%, une fois avec $NaHCO_3$ saturé et une fois à l'eau. On a séché, concentré et distillé au four à boules sur résidu. On a isolé 0,4 g (84% pur) d'un mélange optiquement actif ($[\alpha]^{20}_D$=-18,9°, c=5,7% $CHCl_3$) contenant les deux isomères susmentionnés dans un rapport cis/trans = 60:40.

(-)-(1R,3S)-2,2,3-triméthyl-6-méthylène-1-cyclohexanecarbaldéhyde

IR : 3100, 2970, 2750, 1725, 1650, 1460 $cm^{-1}$

RMN($^1H$,360MHz) : 0,87(d,J = 7Hz,3H) ; 0,98(s,3H) ; 1,00(s,3H) ; 2,54(d, J = 5Hz,1H) ; 4,52(s,1H) ; 4,93(s,1H) ; 9,92(d,J = 5Hz,1H) δ ppm

RMN($^{13}C$) : 15,35(q) ; 15,57(q) ; 27,2(q) ; 31,6(t) ; 36,1(t) ; 38,0(s) ; 41,7(d) ; 65,3(d) ; 109,5(t) ; 145,2(s) ; 205,2(d) δ ppm

SM : 166($M^+$,5), 151(22), 137(18), 133(13), 123(32), 109(46), 95(100), 83(69), 67(31), 55(48), 41(18)

(1S,3S)-2,2,3-triméthyl-6-méthylène-1-cyclohexanecarbaldéhyde

IR : 3100, 2970, 2750, 1725, 1650, 1460 $cm^{-1}$

RMN($^1H$,360MHz) : 0,80(s,3H) ; 0,85(d,3H) ; 1,10(s,3H) ; 2,73(d,J = 4Hz,1H) ; 4,76(s,1H) ; 4,93-(s,1H) ; 9,87(d,J = 4Hz,1H) δ ppm

RMN($^{13}C$) : 14,95(q) ; 21,0(q) ; 26,5(q) ; 31,3(t) ; 32,7(t) ; 37,15(d) ; 37,7(s) ; 69,38(d) ; 113,36(t) ; 143,1(s) ; 205,55(d) δ ppm

SM : 166($M^+$,3), 151(7), 137(30), 123(21), 109(32), 95(100), 83(35), 81(56), 67(27), 55(36), 41(12)

**i**. (+)-(1R,5S)-5-éthyl-2,6,6-triméthyl-2-cyclohexène-1-carbaldéhyde et (1S,5S)-5-éthyl-2,6,6-triméthyl-2-cyclohexène-1-carbaldéhyde

Préparé à partir de 4,7 g (0,026 mole) de l'époxyde optiquement actif décrit à l'Exemple 1g., dans 20 ml de toluène, par addition de 0,03 ml (0,25 mmole) de $SnCl_4$. Après le traitement usuel et distillation au four à boules, on a isolé 4,1 g d'un mélange (76% pur) contenant 82% de l'isomère cis susmentionné et 18% de l'isomère trans correspondant.

Rend. ≈ 87%; $[\alpha]^{20}_D$ = +29,2°

IR : 1675, 1720, 710, 2960 $cm^{-1}$

(+)-(1R,5S)-5-éthyl-2,6,6-triméthyl-2-cyclohexène-1-carbaldéhyde

RMN($^1H$,360MHz) : 0,93(s,3H) ; 1,00(s,3H) ; 1,63(s,3H) ; 2,54(s large,1H) ; 5,69(s large,1H) ; 9,62-(d,J = 5Hz,1H) δ ppm

SM : 180($M^+$,21), 165(9), 151(69), 133(15), 122(42), 109(84), 95(86), 91(25), 81(35), 69(74), 55(39), 41(100)

(1S,5S)-5-éthyl-2,6,6-triméthyl-2-cyclohexène-1-carbaldéhyde

RMN($^1H$,360MHz) : 0,89(s,3H) ; 1,01(s,3H) ; 1,59(s,3H) ; 2,34(d large, J = 5Hz,1H) ; 5,73(s large,1H) ; 9,56(d,J = 5Hz,1H) δ ppm

SM : 180($M^+$,20), 151(71), 149(65), 122(40), 109(80), 95(88), 41(100)

## Exemple 5

### Préparation d'aldéhydes de formule (III) à partir d'alcools (VII)

### Méthode générale

Dans un ballon à 3 cols, équipé d'une bonne agitation, on a placé du chlorochromate de pyridinium (PCC) en suspension dans le dichlorométhane (sec) et introduit goutte à goutte une solution de l'alcool approprié dans le dichlorométhane (sec). On a brassé le mélange à température ambiante pendant une période de 1 à 2 h, et ajouté de l'éther sulfurique. Le mélange a été passé sur une colonne remplie de $SiO_2$ en utilisant comme éluant l'éther. On a ensuite concentré et distillé au four à boules pour obtenir un produit pur.

Les aldéhydes suivants ont été préparés comme suit.

**a**. 2,2,3,6-tétraméthyl-3-cyclohexène-1-carbaldéhyde

Préparé selon la méthode décrite, à partir de 15,0 g (70 mmole) de PCC dans 150 ml de $CH_2Cl_2$ et de 8,38 g (50 mmole) de cis-2,2,3,6-tétraméthyl-3-cyclohexène-1-méthanol, obtenu selon l'Exemple 3b., dans 50 ml de $CH_2Cl_2$. Après un brassage d'1 h et le traitement décrit, on a obtenu 6,77 g de l'aldéhyde désiré sous forme d'une huile jaune (2 isomères, cis/trans ≈ 9:1).

P. éb. : 130°/1,3x10 Pa; rend. 82%

IR : 2990, 1740, 1470 $cm^{-1}$

RMN($^1H$,360MHz) : 0,98(d,J = 7,2Hz,3H) ; 1,00(s,3H) ; 1,14(s,3H) ; 1,72(s large, 3H) ; 1,60-2,30-(m,4H) ; 5,46(s large,1H) ; 9,66(d, J = 6,5Hz,1H) δ ppm

SM : 166($M^+$,11), 151(9), 135(22), 126(56), 108(55), 95(82), 81(100), 67(42), 55(36), 41(13)

**b**. 2,2,6-triméthyl-3-méthylène-1-cyclohexanecarbaldéhyde

Préparé selon la méthode décrite à partir de 13,0 g (0,06 mole) de PCC dans 70 ml de $CH_2Cl_2$ et d'une solution de 6,45 g (0,038 mole) de 2,2,6-triméthyl-3-méthylène-1-cyclohexaneméthanol (obtenu selon l'Exemple 3a.) dans 30 ml de $CH_2Cl_2$. Après 2 h de brassage et le traitement décrit, on a obtenu 5,58 g d'une huile incolore contenant 80% de l'aldéhyde désiré (2 isomères, cis /trans ≈ 9:1) et un produit secondaire non intéressant.

| | |
|---|---|
| P. éb. : | 130°/5x$10^2$ Pa ; rend. 88% |
| IR : | 3100, 2950, 1730, 1650, 1460, 900 $cm^{-1}$ |
| RMN($^1$H,360MHz) : | 0,91(d,J=7,2Hz,3H) ; 1,07(s,3H) ; 1,18(s,3H) ; 1,49(m, 1H) ; 1,76(m,1H) ; 1,93-(t,J=5,4Hz,1H) ; 2,22(m,1H) ; 2,39(m,1H) ; 2,55(m,1H) ; 4,76(s,1H) ; 4,99(s,1H) ; 9,64(d,J=7,2Hz,1H) δ ppm |
| RMN($^{13}$C) : | 207,8(d) ; 152,4(s) ; 108,5(t) ; 64,2(d) ; 32,6(t) ; 32,5(t) ; 30,5(d) ; 28,2(q) ; 26,5-(q) ; 19,6(q) δ ppm |
| SM : | 166($M^+$,2), 160(5), 151(11), 145(21), 135(20), 123(52), 109(35), 95(81), 81(100), 67(41), 55(34), 41(17) |
| Odeur : | fenchylique, moisi, ambrinol |

Exemple 6

Préparation de cétones de formule (I)

Méthode générale

La méthode de préparation des cétones de formule (I) et de leurs formes isomériques racémiques ou optiquement actives à partir des aldéhydes décrits dans les Exemples 4 et 5 peut être illustrée comme suit :

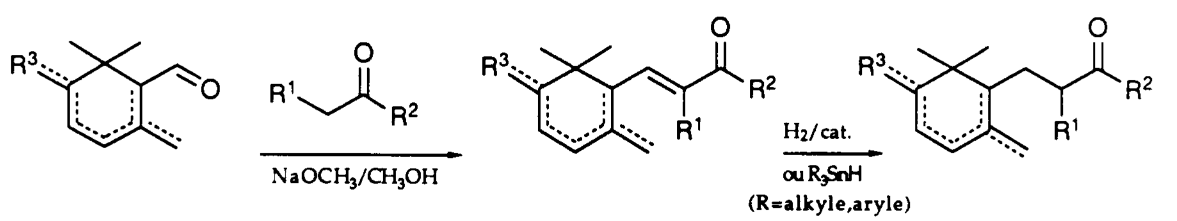


La première étape du procédé illustré dans ce schéma consiste en une condensation aldolique qui s'est effectuée dans les conditions suivantes.

Dans un ballon sous azote, on a introduit le carbaldéhyde (III) approprié dans le méthanol ou l'éthanol. On a chauffé à 50° et introduit du méthylate de sodium à 30% dans le méthanol, puis la cétone appropriée. On a laissé réagir ≈ 20 h à cette température (parfois la température devrait être plus ou moins élevée et le temps de réaction plus ou moins long). On a laissé refroidir et neutralisé le mélange de la réaction. Après extraction à l'éther, on a lavé à neutralité, séché, concentré et distillé le produit brut. Le cas échéant, une purification supplémentaire par chromatographie a été effectuée.

La deuxième étape, lorsqu'elle est une hydrogénation classique, s'est déroulée par exemple dans les conditions suivantes.

Dans un ballon muni d'une tête à hydrogénation, on a chargé 1 éq./g de cétone insaturée, 10 éq./g d'acétate d'éthyle et 0,5 g/mole de Pd/C à 10%. On a fait passer un courant d'hydrogène. On a filtré et concentré le produit de la réaction.

Un exemple d'une réaction de réduction à l'aide d'un hydrure de trialkylétain, pouvant constituer cette deuxième étape, est décrit sous l'alinéa p.

Comme il a été mentionné précédemment, les cétones de formule (I) possédant un cycle saturé peuvent également être préparées en 1 pot à partir de la réaction d'un époxyde (II) avec iodure de magnésium, suivie d'une condensation aldolique telle que décrite ci-dessus, sans séparation de l'aldéhyde intermédiaire. Cette méthode est illustrée sous l'alinéa a. On a préparé les cétones suivantes.

**a**. (E)-4-(2,2,c-3,t-6-tétraméthyl-r-1-cyclohexyl)-3-butén-2-one

Ce composé a été préparé en 1 pot, à partir de 4,4,t-5,r-8-tétraméthyl-1-oxaspiro[2.5]octane, obtenu selon l'Exemple 1d.

Dans un ballon sous azote, on a placé 1,3 g (54,2 mmole) de magnésium et 12,8g (50,4 mmole) d'iode dans 60 ml de THF. On a agité pendant 4 h en maintenant la température à 45° et ensuite évaporé le solvant à pression réduite et à la même température. Après rétablissement de la pression à l'aide de $N_2$, on a introduit 90 ml de toluène sec, porté à reflux et ajouté 60 g (357 mmole) de l'époxyde susmentionné. Après 1 h 1/2, on a ajouté lentement 100 ml d'une solution à 30% de $NaOCH_3$ dans le méthanol et laissé refroidir à 50°. On a ajouté 60 ml (817 mmole) d'acétone et agité pendant 15 h (1 nuit). Après refroidissement à 5°, on a dilué avec 100 ml de toluène, neutralisé à l'aide d'une solution aqueuse à 15% de HCl, séparé, extrait la phase aqueuse à l'aide de 15 ml de toluène, séché sur $MgSO_4$ et concentré. La distillation sur résidu a permis d'obtenir 49,4 g de l'énone désirée pure à 89%.
Une distillation sur colonne Vigreux (12 cm) de ce produit a fourni 24 g d'un produit pur à 97,4%.

| | |
|---|---|
| P. éb. : | 62°/1,33 Pa ; rend. 56% |
| IR : | 2980, 1670, 1620, 1450, 1355, 1250 $cm^{-1}$ |
| RMN($^1$H,360MHz) : | 0,74(d,J=7Hz,3H) ; 0,76(s,3H) ; 0,83(s,3H) ; 0,85(d,J=7Hz, 3H) ; 2,26(s,3H) ; 6,30(d,J=16Hz,1H) ; 6,62(dd,$J_1$=16Hz, $J_2$=10Hz,1H) δ ppm |
| SM : | 208($M^+$,10), 193(6), 175(8), 165(14), 150(19), 111(43), 95(31), 81(31), 55(40), 43(100) |

L'un des énantiomères optiquement actifs de cette cétone, à savoir la (+)-(1R,3S,6S)-4-(2,2,3,6-tétraméthyl-1-cyclohexyl)-3-butén-1-one, a été préparée par condensation aldolique, avec l'acétone, de l'aldéhyde optiquement actif décrit dans l'Exemple 4b. Cette cétone optiquement active, qui contenait 6% de son diastéréomère (1S,3S,6S), possédait un $[\alpha]^{20}_D$= +28,15°, c=0,6% $CHCl_3$.

Odeur : décrite dans l'introduction.

**b**. (E)-4-méthyl-1-(2,2,c-3,t-6-tétraméthyl-r-1-cyclohexyl)-1-pentén-3-one Préparée selon la méthode générale de condensation aldolique décrite, à partir de 16 g (0,07 mole) de 2,2,c-3,t-6-tétraméthyl-r-1-cyclohexanecarbaldéhyde (obtenu selon l'Exemple 4b.) et 20g (0,023 mole) d'isopropylméthyl cétone. Après distillation sur un pont, on a isolé deux fractions comme suit :
fraction ① 4,2g ≈ 88% de l'énone désirée
fraction ② 7,9 g ≈ 96% de l'énone désirée

| | |
|---|---|
| P. éb. : | 53°/5 Pa ; rend. ≈ 64% |
| IR : | 1625, 1670, 1695 $cm^{-1}$ |
| RMN($^1$H,360MHz) : | 0,73(d,J ≈ 7Hz,3H) ; 0,76(s,3H) ; 0,82(s,3H) ; 0,85(d,J ≈ 7Hz, 3H) ; 1,13-(d,J=7Hz, 6H) ; 2,86(hept,J=7Hz,1H) ; 6,09(d,J ≈ 15Hz,1H) ; 6,68(dd, $J_1$=15Hz,$J_2$=8Hz,1H) δ ppm |
| SM : | 236($M^+$,2), 193(30), 165(14), 150(25), 139(41), 123(34), 109(83), 95(100), 81-(70), 69(47), 55(77), 43(61) |
| Odeur : | boisée, irones. |

**c**. 4-méthyl-1-(2,2,c-3,t-6-tétraméthyl-r-1-cyclohexyl)-3-pentanone
Préparée par hydrogénation de l'énone décrite sous b. (2,7 g, 0,01 mole). Après distillation sur bande tournante, on a obtenu 3,3 g de la pentanone susmentionnée pure à 95%.

| | |
|---|---|
| P. éb. : | 53°/10,6 Pa ; rend. ≈ 87% |
| IR : | 1710 $cm^{-1}$ (C=0) |
| RMN($^1$H,360MHz) : | 0,66(s,3H) ; 0,81(d,J ≈ 7Hz,3H) ; 0,87(d,J ≈ 7Hz,3H) ; 0,88(s,3H) ; 1,10(d, J=7Hz,6H) ; 2,40(m,1H) ; 2,55(m,2H) δ ppm |
| SM : | 238($M^+$,10), 195(15), 177(31), 152(33), 137(43), 121(30), 109(48), 97(65), 83-(100), 71(88), 55(87), 43(95) |
| Odeur : | camphrée, légèrement boisée. |

**d**. (E)-2-méthyl-1-(2,2,c-3,t-6-tétraméthyl-r-1-cyclohexyl)-1-pentén-3-one
Préparée par condensation aldolique de 16,8 g (0,1 mole) de 2,2,c-3,t-6-tétraméthyl-r-1-cyclohexanecarbaldéhyde (voir Exemple 4b.) et 25,8 g (0,3 mole) de 3-pentanone. On a chauffé pendant 90 h à 65°. Après distillation sur une colonne Vigreux (10 cm), on a isolé 9 g de l'énone désirée pure à 80%.

| | |
|---|---|
| P. éb. : | 49°/3,5 Pa; rend. ≈ 36% ; P. f. 45-51° |
| IR : | 1640-1660 $cm^{-1}$ (C=0) |
| RMN($^1$H,360MHz) : | 0,72(d, J ≈ 7Hz,3H); 0,77(s,3H) ; 0,80(s,3H) ; 0,85(d,J ≈ 6Hz, 3H) ; 1,12(t,J ≈ 7Hz,3H) ; 1,79(s,3H) ; 2,42(q,J=7Hz,2H) ; 6,50(d,J ≈ 11Hz, 1H) δ ppm |
| SM : | 236($M^+$,8), 193(11), 150(27), 137(100), 123(90), 109(68), 99(23), 95(63), 83(48), 69(25), 57(61), 43(32) |
| Odeur : | boisée, assez faible. |

**e**. (E)-4-méthyl-1-(2,2,c-3,t-6-tétraméthyl-r-1-cyclohexyl)-1-hexén-3-one
Préparée par condensation aldolique de 15,0 g (89 mmole) de 2,2,c-3,t-6-tétraméthyl-r-1-cyclohexanecarbaldéhyde dans 25 ml de méthanol et 20,5 g (205 mmole) de 3-méthyl-2-pentanone. La réaction s'est déroulée à 50° et 41,3 ml (223 mmole) de méthylate de sodium 5,4M dans le méthanol ont été utilisés. Après le traitement usuel et distillation sur colonne Vigreux (20 cm), on a obtenu 17,1 g d'un mélange pur à 94% contenant 93% de l'hexénone susmentionnée et 7% de son isomère (t-3,c-6).

| | |
|---|---|
| P. éb.: | 75°/1 Pa; rend. ≈ 72% |
| IR : | 2960, 2940, 1700, 1625, 1460, 1200, 1000 $cm^{-1}$ |
| RMN($^1$H,360MHz) : | 0,73(d,J=7Hz,3H) ; 0,76(s,3H) ; 0,82(s,3H) ; 0,85(d, J=7Hz,3H) ; 0,89-(t,J=7Hz,3H) ; 1,1(d,J=7Hz,3H) ; 0,9-1,75(m,9H) ; 2,7(m,1H) ; 6,1-(d,J=18Hz,1H) ; 6,68(dd,$J_1$=8Hz,$J_2$=18Hz,1H) δ ppm |
| SM : | 250($M^+$,4), 207(6), 193(33), 153(26), 123(30), 109(83), 95(100), 81(65), 57(81) |

isomère t-3,c-6

| | |
|---|---|
| SM : | 250($M^+$,2), 207(4), 193(28), 151(29), 123(29), 109(71), 95(92), 81(60), 67(43), 55(100) |
| Odeur : | moisie, fleurie, ambrinol. |

**f.** (E)-1-(2,2,c-3,t-6-tétraméthyl-r-1-cyclohexyl)-1-pentén-3-one

Préparée par condensation aldolique de 16,8 g (0,1 mole) de 2,2,c-3,t-6-tétraméthyl-r-1-cyclohexanecarbaldéhyde et 22 g (0,3 mole) de méthyléthyl cétone. Après distillation des têtes (2,2 g) au vide humide et distillation sur résidus, on a obtenu deux fractions, comme suit :

fraction ① 9,2 g de l'énone désirée pure à 88%
fraction ② 3,3 g de l'énone désirée pure à 94%

| | |
|---|---|
| P. éb. : | 60°/5 Pa ; rend. ≈ 70% |
| IR : | 1620-1670 $cm^{-1}$ (C=0) |
| RMN($^1$H,360MHz) : | 0,73(d,J≈7Hz,3H) ; 0,75(s,3H) ; 0,82(s,3H) ; 0,84(d, J≈7Hz,3H) ; 1,11-(t,J≈7Hz,3H) ; 2,58(q,J=7Hz,2H) ; 6,04(d,J≈15Hz,1H) ; 6,64-(dd,$J_1$=15Hz,$J_2$=8Hz,1H) δ ppm |
| SM : | 222($M^+$,6), 193(11), 179(11), 165(12), 150(32), 138(27), 123(98), 109(57), 95-(79), 81(100), 69(30), 55(99), 41(49) |

isomère t-3,t-6

| | |
|---|---|
| SM : | 222($M^+$,6), 193(18), 175(14), 165(11), 150(35), 138(27), 123(100), 109(48), 95(65), 81(65), 69(27), 55(52) |
| Odeur : | moisie, terreuse, humus. |

**g**. (E)-4-(2,2,t-3,t-6-tétraméthyl-r-1-cyclohexyl)-3-butén-2-one

Ce composé a été préparé par condensation aldolique à partir de 1,5 g (9 mmole) de 2,2,t-3,t-6-tétraméthyl-1-cyclohexanecarbaldéhyde [préparé selon la méthode décrite par K.H. Schulte-Elte et al. dans Helv. Chim. Acta 68, 1961 (1985)] dans 10 ml de méthanol, 2,5 ml de méthylate de sodium 5,4 M dans le méthanol et 2 ml d'acétone. Après 24 h de réaction, on a ajouté de nouveau 2,5 ml de méthylate de sodium 5,4 M et 0,7 ml d'acétone. 48 h plus tard, on a extrait et traité le produit de la réaction de la façon usuelle, pour obtenir 2,4 g de produit brut. Ce dernier a été distillé au four à boules (P. éb. 110°/2,1x10 Pa) pour fournir 1,6 g de l'énone désirée pure (rend. 86%).

| | |
|---|---|
| IR : | 2950, 1670, 1620, 1450, 1380, 1250, 905 $cm^{-1}$ |
| RMN($^1$H,360MHz) : | 0,75(d,J=7Hz,3H) ; 0,78(s,3H) ; 0,94(d,J=7Hz,3H) ; 1,0(s,3H) ; 2,26(s,3H) ; 6,3(d,J=18Hz,1H) ; 6,6(dd,$J_1$=18Hz,$J_2$=8Hz,1H) δ ppm |
| RMN($^{13}$C) : | 14,67(q) ; 21,54(q) ; 23,73(q) ; 27,05(q) ; 28,6(q) ; 28,6(t) ; 29,0(t) ; 31,55(d) ; 36,1(s) ; 39,53(d) ; 52,45(d) ; 135,14(d) ; 150,37(d) ; 198,23(s) δ ppm |
| SM : | 208($M^+$,5), 193(5), 175(7), 165(9), 150(19), 137(12), 124(23), 111(70), 95(65), 81(58), 55(47), 42(100) |
| Odeur : | décrite dans l'introduction. |

**h**. (E)-1-(2,2,c-3,t-6-tétraméthyl-r-1-cyclohexyl)-1-heptén-3-one

Préparée par condensation aldolique de 16,6 g (0,1 mole) de 2,2,c-3,t-6-tétraméthyl-r-1-cyclohexanecarbaldéhyde et 23,0 g (0,23 mole) de 2-hexanone. Après distillation sur un pont, on a isolé deux fractions contenant l'hepténone désirée :

fraction ① : 3,9 g pure à 90%
fraction ② : 14,2 g pure à 97%

| | |
|---|---|
| P. éb. : | 62°/3 Pa; rend. 70% |
| IR : | 1625, 1670 $cm^{-1}$ (C=0) |
| RMN($^1$H,360MHz) : | 0,73(d,J≈7Hz,3H) ; 0,755(s,3H) ; 0,82(s,3H) ; 0,85(d, J≈7Hz,3H) ; 0,93-(t,J≈7Hz,3H) ; 2,55(t,J≈7Hz,2H) ; 6,04(d,J≈15Hz, 1H) ; 6,63-(dd,$J_1$=15Hz,$J_2$=8Hz,2H) δ ppm |
| SM : | 250($M^+$,1), 153(32), 135(16), 123(29), 109(71), 95(92), 85(75), 81(100), 67(35), |

55(65), 41(30).

**i**. trans,(E)-4-(2,2,6-triméthyl-3-méthylène-1-cyclohexyl)-3-butén-2-one

Cette énone a été préparée par réaction de 7 g (0,042 mole) de 2,2,3,6-triméthyl-3-méthylène-1-cyclohexanecarbaldéhyde (préparé selon l'Exemple 5b.) dans 15 ml d'éthanol absolu, 9,3 ml de méthylate de sodium (30%) et 0,08 mole d'acétone. On a brassé pendant 24 h à température ambiante. Après le traitement usuel, on a distillé au four à boules (150°/5x10 Pa) et soumis le produit à une chromatographie "Flash" (cyclohexane/éther acétique : 95:5). On a obtenu 2,26 g (rend. 26%) de l'énone susmentionnée à l'état pur.

| | |
|---|---|
| IR : | 3100, 2950, 1660, 1250, 1000, 900 $cm^{-1}$ |
| RMN($^1$H,360MHz) : | 0,77(d,J = 7,2Hz,3H) ; 1,02(s,3H) ; 1,04(s,3H) ; 0,98-1,12(m,1H) ; 1,61(m,1H) ; 1,70-1,90(m,2H) ; 2,19(m,1H) ; 2,27(s,3H) ; 2,37(m,1H) ; 4,67(s,1H) ; 4,69(s,1H) ; 6,04(d,J = 16,2Hz,1H) ; 6,62(dd, $J_1$ = 10,8Hz,$J_2$ = 16,2Hz,1H) δ ppm |
| RMN($^{13}$C) : | 198(s) ; 156,1(s) ; 148,9(d) ; 133,6(d) ; 105,8(t) ; 59,3(d) ; 39,2(s) ; 36,6(t) ; 32,7(t) ; 31,8(d) ; 27,2,(q) ; 26,9(q) ; 21,1(q) ; 21,2(q) δ ppm |
| SM : | 206($M^+$,5), 188(2), 173(7), 163(9), 148(25), 136(20), 121(36), 109(43), 93(43), 81(100), 71(19), 67(41), 55(47), 43(80). |
| Odeur : | décrite dans l'introduction. |

**j**. trans,(E)-1-(2,2,6-triméthyl-3-méthylène-1-cyclohexyl)-1-pentén-3-one

Préparé de façon identique à celle décrite en i., mais en remplaçant l'acétone par la 2-butanone.

| | |
|---|---|
| IR : | 3100, 2950, 1680, 1620, 1460, 1200, 900 $cm^{-1}$ |
| RMN($^1$H,360MHz) : | 0,76(d,J = 7,2Hz,3H) ; 1,02(s,3H) ; 1,04(s,3H) ; 1,12(t,J = 7,2Hz,3H) ; 1,52-1,91-(m,3H) ; 2,19(m,1H) ; 2,37(m,1H) ; 2,59(q, J = 7,2Hz,2H) ; 4,66(s,1H) ; 4,68-(s,1H) ; 6,04(d,J = 15Hz,1H) ; 6,65(dd, $J_1$ = 10Hz,$J_2$ = 15Hz,1H) δ ppm |
| RMN($^{13}$C) : | 200,6(s) ; 156,2(s) ; 147,6(d) ; 132,5(d) ; 105,7(t) ; 59,3(d) ; 39,2(s) ; 36,6(t) ; 33,6(t) ; 32,7(t) ; 31,8(d) ; 26,9(q) ; 22,1(q) ; 21,3(q) ; 8,3(q) δ ppm |
| SM : | 220($M^+$,9), 205(5), 187(9), 173(7), 163(17), 148(37), 136(33), 121(53), 107(64), 81(100), 67(46), 57(82). |
| Odeur : | décrite dans l'introduction. |

**k**. trans,(E)-4-(2,2,3,6-tétraméthyl-3-cyclohexén-1-yl)-3-butén-2-one et cis,(E)-4-(2,2,3,6-tétraméthyl-3-cyclohexén-1-yl)-3-butén-2-one

On a suivi la méthode de préparation décrite en i. mais en utilisant comme produit de départ le 2,2,3,6-tétraméthyl-3-cyclohexène-1-carbaldéhyde obtenu selon l'Exemple 5a. On a obtenu 43 g (rend. 50%) d'un mélange pur contenant 58% de l'isomère trans et 42% de l'isomère cis de l'énone susmentionnée. Ces deux isomères ont ensuite été séparés par chromatographie en phase gazeuse (colonne Carbowax 5m). Leurs données analytiques et qualités olfactives étaient les suivantes :

trans,(E)-4-(2,2,3,6-tétraméthyl-3-cyclohexén-1-yl)-3-butén-2-one

| | |
|---|---|
| IR : | 2950, 1680, 1360, 1250, 1000 $cm^{-1}$ |
| RMN($^1$H,360MHz) : | 0,80(d,J = 7,2Hz,3H) ; 0,95(s,3H) ; 0,98(s,3H) ; 1,59-1,70(m,1H) ; 1,66(s large,3H) ; 1,82(m,2H) ; 2,10(m,1H) ; 2,28(s,3H) ; 5,33(m,1H) ; 6,07(d,J = 16Hz,1H) ; 6,69(dd,$J_1$ = 10,8Hz,$J_2$ = 16Hz,1H) δ ppm |
| RMN($^{13}$C) : | 198,0(s) ; 149,7(d) ; 140,4(s) ; 133,6(d) ; 121,2(d) ; 56,7(d) ; 38,4(s) ; 34,7(t) ; 28,3(d) ; 27,1(q) ; 26,4(q) ; 22,1(q) ; 20,9(q) ; 18,9(q) δ ppm |
| SM : | 206($M^+$,1), 188(1), 163(3), 122(3), 111(26), 96(100), 81(60), 67(6), 55(7), 43(28). |
| Odeur : | décrite dans l'introduction. |

cis,(E)-4-(2,2,3,6-tétraméthyl-3-cyclohexén-1-yl)-3-butén-2-one

| | |
|---|---|
| IR : | 2950, 1670, 1460, 1360, 1260 $cm^{-1}$ |
| RMN($^1$H,360MHz) : | 0,84(d,J = 7,2Hz,3H) ; 0,88(s,3H) ; 1,13(s,3H) ; 1,56-1,67(m,1H) ; 1,66(s large,3H) ; 1,98(m,2H) ; 2,15-2,30(m,1H) ; 2,24(s,3H) ; 5,36(m,1H) ; 6,07-(d,J = 16Hz,1H) ; 6,62(dd,$J_1$ = 10,8Hz, $J_2$ = 16Hz,1H) δ ppm |
| RMN($^{13}$C) : | 198,0(s) ; 148,1(d) ; 139,0(s) ; 133,8(d) ; 121,1(d) ; 56,1(d) ; 37,9(s) ; 31,1(t) ; 29,0(q) ; 28,2(d) ; 26,8(2q) ; 20,0(q) ; 19,0(q) δ ppm |
| SM : | 206($M^+$,2), 149(3), 136(4), 121(9), 111(18), 96(100), 91(100), 81(78), 67(9), 55-(9), 43(12). |
| Odeur : | irone, violette, légèrement fruitée. En fond, lactonique, sèche. |

**l**. trans,(E)1-(2,2,3,6-tétraméthyl-3-cyclohexén-1-yl)-1-pentén-3-one et cis,(E)-1-(2,2,3,6-tétraméthyl-3-cyclohexén-1-yl)-1-pentén-3-one

On a utilisé la méthode décrite en k. en utilisant la 2-butanone comme réactif. On a obtenu 4,8 g d'un mélange pur contenant 60% de l'isomère trans et 40% de l'isomère cis de la penténone susmentionnée.

Ces deux isomères ont été séparés comme décrit en k. Leurs données analytiques et qualités olfactives étaient les suivantes :

trans,(E)-1-(2,2,3,6-tétraméthyl-3-cyclohexén-1-yl)-1-pentén-3-one

| | |
|---|---|
| IR : | 2970, 1680, 1460, 1360, 1200, 1000 $cm^{-1}$ |
| RMN($^1$H,360MHz) : | 0,80(d,J = 6,5Hz,3H) ; 0,93(s,3H) ; 0,97(s,3H) ; 1,13(t, J = 7,2Hz,3H) ; 1,58-1,71-(m,1H) ; 1,66(s large,3H) ; 1,80(m,2H) ; 2,10(m,1H) ; 2,60(q,J = 7,2Hz,2H) ; 5,37(m,1H) ; 6,08(d,J = 15,5Hz,1H) ; 6,72(dd, $J_1$ = 10,8Hz,$J_2$ = 15,5Hz,1H) δ ppm |
| RMN($^{13}$C) : | 200,6(s) ; 148,4(d) ; 140,4(s) ; 132,5(d) , 121,2(d) ; 56,7(d) ; 38,4(s) ; 34,7(t) ; 33,4(t) ; 28,3(d) ; 26,4(q) ; 22,1(q) ; 21,0(q) ; 19,0(q) δ ppm |
| SM : | 220($M^+$,0), 163(2), 148(2), 125(18), 107(5), 96(100), 91(6), 81(67), 67(9), 57(12), 41(6). |
| Odeur : | myrrhe, olibanum, sesquiterpène, bisabolène. |

cis,(E)-1-(2,2,3,6-tétraméthyl-3-cyclohexén-1-yl)-1-pentén-3-one

| | |
|---|---|
| IR : | 2950, 1670, 1620, 1460, 1360, 1200, 1000 $cm^{-1}$ |
| RMN($^1$H,360MHz) : | 0,85(d,J = 7,2Hz,3H) ; 0,88(s,3H) ; 1,10(t,J = 7,2Hz,3H) ; 1,14(s,3H) ; 1,60-1,70-(m,1H) ; 1,64(s large,3H) ; 1,92-2,03(m,2H) ; 2,20(m,1H) ; 2,57(q,J = 7,2Hz, 2H) ; 5,35(m,1H) ; 6,09(d,J = 15,5Hz,1H) ; 6,65(dd, $J_1$ = 10,8Hz,$J_2$ = 15,5Hz,1H) δ ppm |
| RMN($^{13}$C) : | 200,9(s) ; 146,7(d) ; 139,0(s) ; 132,7(d) ; 121,2(d) ; 56,1(d) ; 37,7(s) ; 33,1(t) ; 31,2(t) ; 29,0(q) ; 28,2(d) ; 26,8(q) ; 20,0(q) ; 19,0(q) ; 8,3(q) δ ppm |
| SM : | 220($M^+$,2), 205(1), 191(1), 163(3), 148(1), 135(3), 125(15), 107(10), 96(100), 81-(68), 67(10), 57(19), 41(7). |
| Odeur : | myrrhe, résineuse. |

**m**. trans,(E)-4-(2,5,6,6-tétraméthyl-2-cyclohexén-1-yl)-3-butén-2-one (trans-α-irone), cis,(E)-4-(2,5,6,6-tétraméthyl-2-cyclohexén-1-yl)-3-butén-2-one (cis-α-irone) et (E)-4-(2,5,6,6-tétraméthyl-1-cyclohexén-1-yl)-3-butén-2-one (β-irone)

Un mélange des trois irones susmentionnées a été préparé à partir d'un mélange des aldéhydes décrits dans l'Exemple 4, f. et g. Ce mélange de départ (19 g, 114,4 mmole), contenant 48% de trans-2,5,6,6-tétraméthyl-2-cyclohexène-1-carbaldéhyde, 43% de l'isomère cis correspondant et 9% de 2,5,6,6-tétraméthyl-1-cyclohexène-1-carbaldéhyde, dissous dans 10 ml d'éthanol, a été soumis à une condensation aldolique, selon la méthode générale décrite, avec 20 ml d'acétone (272,4 mmole) et 1 ml de $NaOCH_3$ à 30% dans le méthanol (≈ 5,5 mmole). Après 48 h de réaction à température ambiante et le traitement usuel de la phase organique, on a obtenu 28,3 g d'une huile jaune, qui a été purifiée par distillation sur une colonne Vigreux de 12 cm pour fournir trois fractions comme suit :

| | |
|---|---|
| tête : | 2,4 g d'un mélange (81% pur) contenant 52% de β-irone, 43% de trans-α-irone et 5% de cis-α-irone |
| coeur : | 13,3 g d'un mélange (97% pur) contenant 78% de β-irone, 14% de trans-α-irone et 5% de cis-α-irone |
| queue : | 3,79% de β-irone pur à 94%. |

D'autre part, lorsqu'on a ajouté une solution de 3,0 g (19,74 mmole) de 2,5,6,6-tétraméthyl-1-cyclohexène-1-carbaldéhyde pur dans 3 ml d'éthanol et 3 ml d'acétone (41 mmole) à 4 ml (≈ 22 mmole) d'une solution de $NaOCH_3$ à 30% dans le méthanol, laissant réagir pendant 18 h à température ambiante, on a obtenu, après le traitement usuel et distillation au four à boules, un mélange (3,29 g) contenant 34% de trans-α-irone, 6% de cis-α-irone et 50% de β-irone (rend. 76%):

trans-α-irone

SM : 206($M^+$,7), 191(4), 136(51), 121(100), 109(13), 93(68), 43(60)

cis-α-irone

SM : 206($M^+$,8), 191(4), 136(50), 121(100), 109(12), 93(68), 43(53)

β-irone

| | |
|---|---|
| IR : | 2900, 1660, 1440, 1355, 1245, 780 $cm^{-1}$ |
| RMN($^1$H,360MHz) : | 0,91(d,J = 7Hz,3H) ; 0,91(s,3H) ; 1,06(s,3H) ; 1,73(s,3H) ; 2,30(s,3H) ; 6,07-(d,J = 16Hz,1H) ; 7,25(d,J = 16Hz,1H) δ ppm |
| SM : | 206($M^+$,4), 191(100), 149(15), 121(18), 43(52). |

**n**. (E)-1-(2,5,6,6-tétraméthyl-1-cyclohexén-1-yl)-1-pentén-3-one et (E)-1-(2,5,6,6-tétraméthyl-2-cyclohexén-1-yl)-1-pentén-3-one

Ces composés ont été préparés à partir de 5 g (0,03 mole) de trans-2,5,6,6-tétraméthyl-2-cyclohexène-1-carbaldéhyde (obtenu selon l'Exemple 4f.) et de 5,2 g (0,72 mole) de butanone dans 20 ml d'éthanol. On a ajouté à ce mélange 0,3 ml de méthylate de sodium à 30% dans le méthanol et chauffé à reflux

pendant 96 h. Après le traitement usuel et distillation sur un pont, on a obtenu 2,6 g d'un mélange pur à 95% contenant 66,8% de (E)-1-(2,5,6,6-tétraméthyl-1-cyclohexén-1-yl)-1-pentén-3-one, 27,1% de trans,-(E)-1-(2,5,6,6-tétraméthyl-2-cyclohexén-1-yl)-1-pentén-3-one et 6,1% de cis,(E)-1-(2,5,6,6-tétraméthyl-2-cyclohexén-1-yl)-1-pentén-3-one.

P. éb. : 75°/5 Pa; rend. 39%

IR : 1600, 1670 $cm^{-1}$ (C=0)

RMN($^1$H,360MHz) :

isomère $\beta$ : 0,91(s,3H) ; 0,91(d,J=7Hz,3H) ; 1,05(s,3H) ; 1,14(t,J=7Hz, 3H) ; 1,73(s,3H) ; 2,61(q,J=7Hz,3H) ; 6,09(d,J=15Hz, 1H) ; 7,28(d,J=15Hz,1H) $\delta$ ppm

isomère $\alpha$-trans : 5,46(s large,1H) ; 6,05(d,J=15Hz,1H) ; 6,71(dd, $J_1$ = 15Hz, $J_2$ = 8Hz,1H) $\delta$ ppm

isomère $\alpha$-cis : 5,52(s large,1H) ; 6,13(d,J=15Hz,1H) $\delta$ ppm

SM : isomère $\beta$ : 220($M^+$,4), 205(100), 163(15), 149(22), 136(15), 121(33), 107(22), 91(27), 77(18), 69(15), 65(10), 57(90), 41(26)

isomère $\alpha$-trans : 220($M^+$,8), 150(44), 135(40), 121(100), 107(10), 93(62), 77(23), 57(52), 41(38)

isomère $\alpha$-cis : 220($M^+$,9), 205(10), 150(53), 135(42), 121(100), 107(10), 93(57), 77(18), 57(30), 41(11).

Odeur : méthylionone, plus ionone qu'irone.

**o**. (-)-(5R,E)-4-(5-éthyl-2,6,6-triméthyl-1-cyclohexène-1-yl)-3-butén-2-one

Ce composé a été préparé par condensation aldolique de 2 g (0,011 mole) de (+)-(1R,5S)-5-éthyl-2,6,6-triméthyl-2-cyclohexène-1-carbaldéhyde (obtenu selon l'Exemple 4i.) avec 2 ml (0,03 mole) d'acétone, dans 10 ml d'éthanol. On a laissé réagir, après l'addition de 0,1 ml de $CH_3ONa$ à 30% dans le méthanol, pendant environ 65 h. Après le traitement usuel, on a isolé 1,3 g d'un mélange pur à 95% contenant 83% de la buténone susmentionnée, 13% de son isomère $\alpha$-trans et 4% de son isomère $\alpha$-cis.

Rend. ≈ 56%

$[\alpha]^{20}_D$ = -5,6°

IR : 1600, 1670 $cm^{-1}$ (C=0)

RMN($^1$H,360MHz) :

isomère $\beta$ : 0,90(s,3H) ; 0,94(t,J≈4Hz,3H) ; 1,07(s,3H) ; 1,74(s large, 3H) ; 2,30(s,3H) ; 6,07(d,J=15Hz,1H) ; 7,25(d,J=15Hz,1H) $\delta$ ppm

isomère $\alpha$-trans : 1,56(s,3H) ; 2,26(s,3H) ; 5,50(s large,1H) ; 6,03-(d,J=15Hz, 1H) ; 6,68(dd,$J_1$ = 15Hz,$J_2$ = 8Hz,1H) $\delta$ ppm

isomère $\alpha$-cis : 5,55(s large,1H) d ppm

SM : isomère $\beta$ : 220($M^+$,5), 205(100), 149(15), 135(10), 121(15), 43(57)

isomère $\alpha$-trans : 220($M^+$,21), 136(69), 121(100), 109(12), 93(78), 77(13), 69(14), 55(18), 43(99)

isomère $\alpha$-cis : 220($M^+$,21), 136(100), 121(99), 93(68), 43(60).

Odeur : ionones.

**p**. 4-méthyl-1-(2,2,c-3,t-6-tétraméthyl-r-1-cyclohexyl)-3-hexanone

Ce composé a été préparé par réduction de l'hexénone décrite en e., selon la méthode suivante.

Dans un ballon à 4 cols, équipé d'un agitateur magnétique, on a chargé 11,1 g (44 mmole) de l'hexénone susmentionnée, 500 ml de toluène et 40,15 g (115 mmole) d'hydrure de triphénylétain et le mélange a été chauffé à reflux pendant 6 jours (141 h). On a laissé refroidir, filtré, concentré et distillé sur résidu pour obtenir 11,1 g d'hexanone pure à 96%.

P. éb. 77°/0,5 Pa ; rend. : 95%

IR : 2960, 1710, 1460, 1370 $cm^{-1}$

RMN($^1$H,360MHz) : 0,5(m,1H) ; 0,66(s,3H) ; 0,82(d,3H) ; 0,87(t,J=7Hz,3H) ; 0,88-(d,J=7Hz,3H) ; 0,89(s,3H) ; 1,06(s,3H) ; 1,0-1,4(m,6H) ; 1,65(m,4H) ; 2,45(m,3H) $\delta$ ppm

SM : 252($M^+$,7), 195(10), 177(20), 152(28), 137(29), 121(20), 109(26), 97(40), 83(63), 69(46), 57(100), 41(53)

Odeur : boisée, camphrée, celluloïde, lavande.

L'hydrure de triphénylétain a été préparé ainsi : dans un ballon à 4 cols, équipé d'un agitateur magnétique, on a chargé 600 ml d'éther et 3,1 g (81 mmole) de $LiAlH_4$ et refroidi à 0° (bain de glace + sel). On a introduit 62,6 g (0,16 mole) de chlorure de triphénylétain à l'aide d'une ampoule d'introduction de poudres (aucune exothermie). On a laissé revenir à température ambiante et agité pendant 1 h. Le

mélange de la réaction a été versé sur glace, extrait à l'éther, lavé 3 fois à l'eau, séché et concentré pour fournir 56,8g de produit brut qui a été utilisé tel quel dans la synthèse ci-dessus.

Exemple 7

Composition parfumante

On a préparé une composition parfumante de base pour un parfum de type féminin, en mélangeant les ingrédients suivants :

| Ingrédients | Parties en poids |
|---|---|
| Florol ® 1) | 200 |
| Acétate de benzyle | 40 |
| Acétate de styrallyle | 120 |
| Acétate de vétyvéryle | 560 |
| 10-Undécénal à 10%* | 80 |
| Aldéhyde hexylcinnamique | 80 |
| Ambrettolide 2) à 10%* | 100 |
| Allyle ionone à 1%* | 80 |
| γ-Undécalactone à 1%* | 160 |
| Cétone framboise à 1%* | 80 |
| Cassis Bourgeons absolue | 100 |
| Essence de coumarine à 10%* | 140 |
| Cyclosia ® 3) base | 500 |
| Essence de galbanum | 60 |
| Essence de clous de girofle | 20 |
| Isobutylquinoléine 4) à 10%* | 40 |
| Isobutyrate de phénoxyéthyle | 20 |
| Jasmin absolue | 240 |
| Levocitrol 5) | 440 |

| | |
|---|---|
| Lilial ® 6) | 40 |
| Ethyl linalol | 140 |
| Lyral ® 7) | 260 |
| Iralia ® 8) | 460 |
| Mousse de chêne absolue à 50%* | 320 |
| Hédione ® 9) | 1140 |
| Essence de néroli | 120 |
| Essence de patchouli | 40 |
| Alcool phényléthylique | 760 |
| Essence d'orange douce du Portugal | 80 |
| Polywood ® 10) | 160 |
| Rose absolue du Maroc | 120 |
| Salicylate de benzyle | 440 |
| Essence de santal | 260 |
| Stemone ® 11) | 60 |
| Tonalid ® 12) à 10%* | 130 |
| Vanilline à 1%* | 50 |
| Vertofix coeur 13) | 1640 |
| Ylang extra | 140 |
| Jacinthe absolue synthétique | 160 |
| Rose absolue synthétique | 320 |
| Total | 9900 |

* dans le dipropylène glycol (DIPG)

1) tétrahydro-2-isobutyl-4-méthyl-4(2H)-pyranol ; origine : Firmenich SA, Genève, Suisse

2) 7-hexadécén-16-olide ; origine : L. Givaudan, Genève, Suisse

3) hydroxycitronellal ; origine : Firmenich SA, Genève, Suisse

4) 6-(1-méthylpropyl)quinoléine ; origine : International Flavours and Fragrances Inc., USA

5) levo-citronellol ; origine : Firmenich SA, Genève, Suisse

6) 3-(4-tert-butyl-1-phényl)-2-méthylpropanal ; origine : L. Givaudan, Genève, Suisse

7) 4-(4-hydroxy-4-méthylpentyl)-3-cyclohexène-1-carboxaldéhyde ; origine : International Flavors & Fragrances Inc., USA

8) méthylionone ; origine : Firmenich SA, Genève, Suisse

9) dihydrojasmonate de méthyle ; origine : Firmenich SA, Genève, Suisse
10) acétate de perhydro-5,5,8a-triméthyl-2-naphtyle ; origine : Firmenich SA, Genève, Suisse
11) 5-méthyl-3-heptanone oxime ; origine : L. Givaudan, Genève, Suisse
12) 7-acétyl-1,1,3,4,4,6-hexaméthyltétraline ; origine : PFW, Hollande
13) origine : International Flavors & Fragrances Inc., USA

A cette composition de base de type fleuri, rosé, jasminé, Chypre, on a ajouté 1% de (E)-4-(2,2,c-3,t-6-tétraméthyl-r-1-cyclohexyl)-3-butén-2-one. On a ainsi obtenu une composition nouvelle possédant une note odorante irisée et balsamique, très poudrée et beaucoup plus arrondie que celle de la composition de base. La note irisée de la composition nouvelle était aussi plus orientale en fond, le composé de l'invention susmentionné ayant apporté un effet olfactif rappelant celui des essences de myrrhe de meilleure qualité.

Exemple 8

Composition parfumante

On a préparé une composition parfumante de base pour un parfum de type masculin, par mélange des ingrédients suivants :

| Ingrédients | Parties en poids |
|---|---|
| Acétate de benzyle | 150 |
| Acétate de lynalyle | 500 |
| Aldéhyde C10 à 10%* | 150 |
| Bergamote synthétique | 600 |
| Citral pur | 500 |
| Essence de citron | 1300 |
| Exaltex ® 1) | 500 |
| Essence de géranium Bourbon | 550 |
| Essence de lavandin | 1000 |
| Lilial ® 2) | 200 |
| Essence de coumarine | 200 |
| Ethyl linalol | 500 |
| Musc ambrette | 500 |
| Oxyde de rose 3) à 10%* | 50 |
| Essence de patchouli | 850 |
| Essence de petitgrain | 150 |
| Acétate de cédryle | 600 |
| Propionate de lynalyle | 150 |
| Salicylate d'amyle | 100 |
| Salicylate de benzyle | 400 |
| Polysantol ® 4) à 10%* | 50 |
| Vertofix coeur 5) | 700 |
| Total | 9700 |

* dans le DIPG
1) pentadécanolide ; origine : Firmenich SA, Genève, Suisse
2) voir Exemple 8, alinéa 6)
3) méthylpropényle méthyle tétrahydropyran ; origine : Firmenich SA, Genève, Suisse
4) (1'R,E)-3,3-diméthyl-5-(2',2',3'-triméthyl-3'-cyclopentén-1'-yl)-4-pentén-2-ol ; origine : Firmenich SA, Genève, Suisse
5) voir Exemple 8, alinéa 13)

A cette composition de base de type aromatique, boisé, on a ajouté à raison de 0,3% en poids le composé selon l'invention cité dans l'exemple précédent. La nouvelle composition ainsi obtenue dégageait une odeur dont la note boisée s'est trouvée nettement adoucie par une note florale-violette, évoquant l'effet odorant de la feuille de violette naturelle. Par ailleurs, le côté hespéridé-frais ressortait beaucoup plus que dans la composition de base, de sorte que la nouvelle composition possédait une connotation plus moderne.

Exemple 9

Test de substantivité sur linge

A un adoucissant textile commercial on a ajouté 0,1% en poids des ingrédients parfumants indiqués ci-après, pour préparer quatre échantillons d'un adoucissant textile parfumé A, B, C et D :

| Echantillon | Ingrédient parfumant |
|---|---|
| A | α-ionone |
| B | α-méthylionone |
| C | α-irone |
| D | (E)-4-(2,2,c-3,t-6-tétraméthyl-r-1-cyclohexyl)-3-butén-2-one |

Dans quatre machines à laver le linge, on a traité séparément quatre lots de textiles mixtes standard, contenant des textiles en coton, en fibre acrylique et en nylon, avec respectivement les échantillons A, B, C et D. Les quatre lots de textiles ainsi traités ont été ensuite évalués à l'aveugle par un panel d'experts

parfumeurs, aussi bien à l'état humide qu'après leur séchage.
Le lot de linge traité avec l'échantillon D dégageait, à l'état humide, une odeur dont l'impact était beaucoup plus fort que celui de l'odeur des trois autres lots de textiles.
Les résultats de ces évaluations comparatives ont montré que, de l'avis unanime des parfumeurs, le lot de textiles traité avec l'échantillon D, contenant la (E)-4-(2,2,c-3,t-6-tétraméthyl-r-1-cyclohexyl)-3-butén-2-one, exhalait une odeur beaucoup plus puissante à la sortie de la machine à laver que l'odeur d'aucun des trois autres lots de textiles. Par ailleurs, l'odeur du lot traité avec l'échantillon D était aussi plus durable après le séchage du linge. Ces résultats démontrent que le composé selon l'invention possède une note odorante d'une rare substantivité, puisque les trois autres ingrédients parfumants susmentionnés sont déjà réputés pour la substantivité de leur note olfactive.

## Revendications

1. Composés de formule

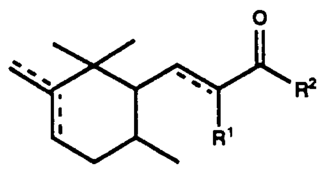

(Ia)

dans laquelle les lignes pointillées indiquent l'emplacement d'une liaison simple ou double, le symbole $R^1$ représente un atome d'hydrogène ou un radical méthyle, le symbole $R^2$ représente un radical alkyle de $C_1$ à $C_4$, saturé ou insaturé, de chaîne linéaire ou ramifiée, à l'exclusion de la 4-(2,2,3,6-tétraméthyl-1-cyclohexyl)-2-butanone, la 4-(2,2,3,6-tétraméthyl-1-cyclohexyl)-3-butén-2-one, la 1-(2,2,3,6-tétraméthyl-1-cyclohexyl)-1-hexén-3-one et de la 1-(2,2,3,6-tétraméthyl-1-cyclohexyl)-3-hexanone.

2. Un composé selon la revendication 1, essentiellement sous une forme isomérique trans de formule

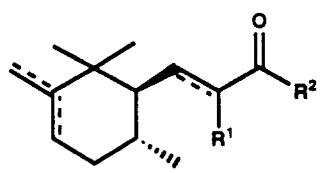

(Ib)

dans laquelle les lignes pointillées dans le cycle indiquent l'emplacement d'une liaison double, la ligne pointillée dans la chaîne latérale indique l'emplacement d'une liaison simple ou double, le symbole $R^1$ représente un atome d'hydrogène ou un radical méthyle et le symbole $R^2$ représente un radical alkyle de $C_1$ à $C_4$, saturé ou insaturé, de chaîne linéaire ou ramifiée, sous forme racémique ou de l'un des énantiomères optiquement actifs.

3. Un composé selon la revendication 1, essentiellement sous une forme isomérique trans de formule

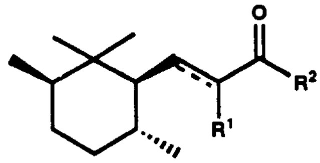

(Ic)

dans laquelle la ligne pointillée indique l'emplacement d'une liaison simple ou double et les symboles $R^1$ et $R^2$ ont le sens indiqué à la revendication 1, sous forme d'un racémate ou de l'un des énantiomères optiquement actifs, à l'exclusion de la 4-(2,2,c-3,t-6-tétraméthyl-r-1-cyclohexyl)-2-butanone, de la 1-(2,2,c-3,t-6-tétraméthyl-r-1-cyclohexyl)-1-hexén-3-one et de la 1-(2,2,c-3,t-6-tétraméthyl-r-1-cyclohexyl)-3-hexanone.

**4.** A titre d'un composé selon la revendication 2, l'un des composés suivants :
a. trans,(E)-4-(2,2,3,6-tétraméthyl-3-cyclohexén-1-yl)-3-butén-2-one
b. trans,(E)-4-(2,2,6-triméthyl-3-méthylène-1-cyclohexyl)-3-butén-2-one
c. trans,(E)-1-(2,2,6-triméthyl-3-méthylène-1-cyclohexyl)-1-pentén-3-one
sous forme d'un racémate ou de l'un de ses énantiomères optiquement actifs.

**5.** A titre d'un composé selon la revendication 3, la (E)-4-(2,2,c-3,t-6-tétraméthyl-r-1-cyclohexyl)-3-butén-2-one sous forme d'un racémate ou de l'un de ses énantiomères optiquement actifs.

**6.** Utilisation d'un composé de formule

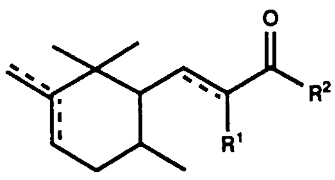

(Ia)

dans laquelle les lignes pointillées indiquent l'emplacement d'une liaison simple ou double, le symbole $R^1$ représente un atome d'hydrogène ou un radical méthyle et le symbole $R^2$ représente un radical alkyle de $C_1$ à $C_4$, saturé ou insaturé, de chaîne linéaire ou ramifiée, à titre d'ingrédient parfumant pour la préparation de compositions parfumantes et articles parfumés.

**7.** Utilisation selon la revendication 6 d'un composé selon la revendication 2.

**8.** Utilisation selon la revendication 6 d'un composé de formule (Ic) telle que définie à la revendication 3, sous forme d'un racémate ou de l'un de ses énantiomères optiquement actifs.

**9.** Utilisation selon la revendication 6 d'un composé selon la revendication 4.

**10.** Utilisation selon la revendication 6 d'un composé selon la revendication 5.

**11.** Composition parfumante résultant de l'utilisation selon l'une des revendications 6 à 10.

**12.** Article parfumé résultant de l'utilisation selon l'une des revendications 6 à 10.

**13.** A titre d'article parfumé selon la revendication 12, un parfum ou une eau de toilette, un savon, un gel de douche ou bain, un shampoing ou autre produit d'hygiène capillaire, une préparation cosmétique, un désodorisant corporel ou d'air ambiant, un détergent ou revitalisant textile, ou un produit d'entretien.

**14.** Procédé pour la préparation d'un composé de formule

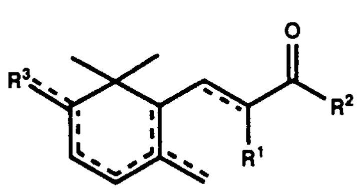

(I)

dans laquelle les lignes pointillées indiquent l'emplacement d'une liaison simple ou double, le symbole $R^1$ représente un atome d'hydrogène ou un radical méthyle, le symbole $R^2$ représente un radical alkyle de $C_1$ à $C_4$, saturé ou insaturé, de chaîne linéaire ou ramifiée et le symbole $R^3$ représente un radical méthyle, éthyle ou méthylène, à l'exclusion de la 4-(2,2,3,6-tétraméthyl-1-cyclohexyl)-2-butanone, la 4-(2,2,3,6-tétraméthyl-1-cyclohexyl)-3-butén-2-one, la 1-(2,2,3,6-tétraméthyl-1-cyclohexyl)-1-hexén-3-one et de la 1-(2,2,3,6-tétraméthyl-1-cyclohexyl)-3-hexanone, le procédé étant caractérisé en ce qu'on :

a. traite un composé de formule

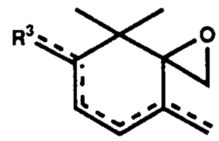

(II)

dans laquelle les lignes pointillées et le symbole $R^3$ sont définis comme ci-dessus, avec un acide de Lewis pour obtenir un aldéhyde de formule

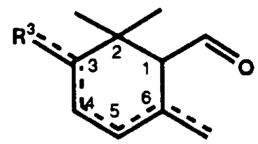

(III)

dans laquelle les lignes pointillées et le symbole $R^3$ ont le sens indiqué plus haut ;
b. transforme, le cas échéant, à l'aide d'une réaction d'isomérisation connue en soi, un aldéhyde de formule (III), possédant une double liaison endocyclique en position 5, en son isomère de formule

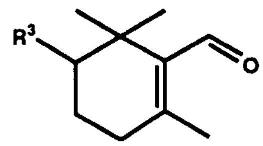

(IIIb)

dans laquelle le symbole $R^3$ a le sens indiqué plus haut ;
c. transforme, à l'aide d'une réaction de condensation aldolique avec une cétone appropriée de formule

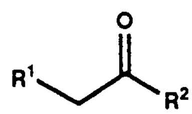

dans laquelle $R^1$ et $R^2$ sont définis comme à la formule (I), l'aldéhyde obtenu selon a. ou b. en un composé de formule (I) possédant une double liaison dans la chaîne latérale ; et,
d. soumet, le cas échéant, ce dernier composé à une réaction d'hydrogénation ou de réduction connue en soi, pour obtenir un composé de formule (I) possédant une chaîne saturée.

**15.** Procédé selon la revendication 14, caractérisé en ce qu'on prépare un composé selon la revendication 1, en utilisant, en tant que produit de départ, un époxyde de formule

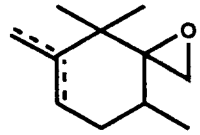

(IIa)

dans laquelle les lignes pointillées ont le sens indiqué à la formule (Ia).

**16.** Procédé selon la revendication 15, caractérisé en ce qu'on prépare un composé selon la revendication 2, en utilisant, en tant que produit de départ, un époxyde essentiellement sous une forme isomérique trans de formule

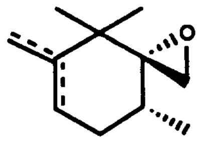

(IIb)

dans laquelle les lignes pointillées sont définies comme à la formule (Ib), ou l'un de ses énantiomères optiquement actifs.

**17.** Procédé selon la revendication 15, caractérisé en ce qu'on prépare un composé selon la revendication 3, en utilisant, en tant que produit de départ, un époxyde essentiellement sous une forme isomérique trans de formule

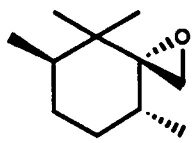

(IIc)

ou l'un de ses énantiomères optiquement actifs.

**18.** Procédé pour la préparation d'un aldéhyde de formule (III) telle que définie à la revendication 14, à l'exclusion du 2,2,3,6-tétraméthyl-1-cyclohexanecarbaldéhyde, caractérisé en ce qu'on traite avec un acide de Lewis un composé de formule (II) telle que définie à la revendication 14.

**19.** Procédé selon la revendication 18, caractérisé en ce qu'on utilise comme produit de départ un composé de formule (IIa) telle que définie à la revendication 15, pour obtenir un ,aldéhyde de formule

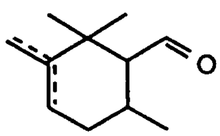

(IIIa)

dans laquelle les lignes pointillées indiquent l'emplacement d'une liaison double.

**20.** Composés de formule (II) telle que définie à la revendication 14, à l'exclusion du 4,4,5,8-tétraméthyl-1-oxaspiro[2.5]octane.

**21.** Composé selon la revendication 20, sous forme d'un racémate de formule (IIb) telle que définie à la revendication 16, ou de l'un de ses énantiomères optiquement actifs.

**22.** Produit selon la revendication 20, sous une forme stéréoisomère racémique de formule

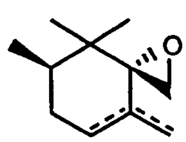

(IId)

dans laquelle les lignes pointillées indiquent l'emplacement d'une liaison double, ou sous forme de l'un des énantiomères optiquement actifs.

**23.** Composé de formule

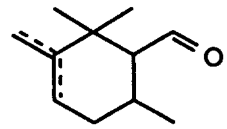

dans laquelle les lignes pointillées indiquent l'emplacement d'une liaison double.

**24.** Composé selon la revendication 23, sous une forme stéréoisomère racémique de formule

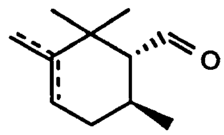

(IIIc)

dans laquelle les lignes pointillées indiquent l'emplacement d'une liaison double, ou sous forme de l'un des énantiomères optiquement actifs.

## Claims

**1.** Compounds of formula

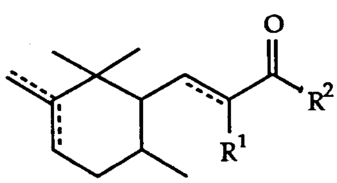

(Ia)

wherein the dotted lines indicate the location of a single or double bond, symbol $R^1$ stands for a hydrogen atom or a methyl radical, symbol $R^2$ represents a linear or branched, saturated or unsaturated, $C_1$ to $C_4$ alkyl radical, with the proviso that 4-(2,2,3,6-tetramethyl-1-cyclohexyl)-3-buten-2-one, 1-(2,2,3,6-tetramethyl-1-cyclohexyl)-1-hexen-3-one and 1-(2,2,3,6-tetramethyl-1-cyclohexyl)-3-hexanone are excluded.

**2.** A compound according to claim 1, essentially as a trans configuration isomer of formula

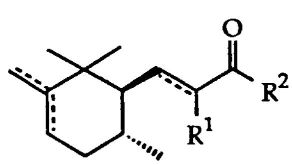

(Ib)

wherein the dotted lines in the ring indicate the location of a double bond, the dotted line in the side chain indicates the location of a single or double bond, symbol $R^1$ stands for a hydrogen atom or a methyl radical and symbol $R^2$ represents a linear or branched, saturated or unsaturated, $C_1$ to $C_4$ alkyl radical, in the form of a racemate or of one of the optically active enantiomers.

**3.** A compound according to claim 1, essentially as a trans configuration isomer of formula

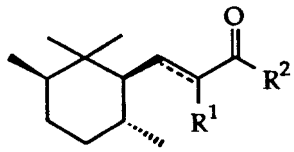

(Ic)

wherein the dotted line indicates the location of a single or double bond and symbols $R^1$ and $R^2$ have the meaning indicated as in claim 1, in the form of a racemate or of one of the optically active enantiomers, with the proviso that 4-(2,2,c-3,t-6-tetramethyl-r-1-cyclohexyl)-2-butanone, 1-(2,2,c-3,t-6-tetramethyl-r-1-cyclohexyl)-1-hexen-3-one and 1-(2,2,c-3,t-6-tetramethyl-r-1-cyclohexyl)-3-hexanone are excluded.

**4.** As a compound according to claim 2, any one of the following compounds :
a. trans, (E)-4-(2,2,3,6-tetramethyl-3-cyclohexen-1-yl)-3-buten-2-one,
b. trans, (E)-4-(2,2,6-trimethyl-3-methylene-1-cyclohexyl)-3-buten-2-one,
c. trans, (E)-1-(2,2,6-trimethyl-3-methylene-1-cyclohexyl)-1-penten-3-one
in the form of a racemate or of one of its optically active enantiomers.

**5.** As a compound according to claim 3, (E)-4-(2,2,c-3,t-6-tetramethyl-r-1-cyclohexyl)-3-buten-2-one in the form of a racemate or of one of its optically active enantiomers.

**6.** Use of a compound of formula

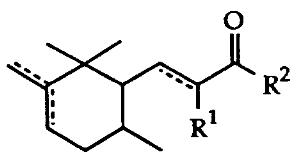

(Ia)

wherein the dotted lines indicate the location of a single or double bond, symbol $R^1$ designates a hydrogen atom or a methyl radical and symbol $R^2$ stands for a linear or branched, saturated or unsaturated, $C_1$ to $C_4$ alkyl radical, as a perfuming ingredient for the preparation of perfuming compositions and perfumed articles.

**7.** Use according to claim 6 of a compound according to claim 2.

**8.** Use according to claim 6 of a compound of formula (Ic) as defined in claim 3, in the form of a racemate or of one of its optically active enantiomers.

**9.** Use according to claim 6 of a compound according to claim 4.

**10.** Use according to claim 6 of a compound according to claim 5.

**11.** Perfuming composition resulting from the use according to any one of claims 6 to 10.

**12.** Perfumed article resulting from the use according to any one of claims 6 to 10.

**13.** As a perfumed article according to claim 12, a perfume or a Cologne, a soap, a shower or bath gel, a shampoo or any other hair care product, a cosmetic preparation, a body or air deodorant, a detergent or a fabric softener or a household product.

**14.** Process for the preparation of a compound of formula

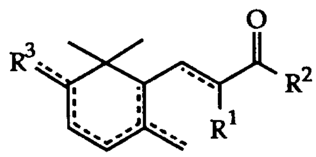

(I)

wherein the dotted lines indicate the location of a single or double bond, symbol $R^1$ designates a hydrogen atom or a methyl radical, symbol $R^2$ represents a linear or branched, saturated or unsaturated, $C_1$ to $C_4$ alkyl radical, and symbol $R^3$ stands for a methyl, ethyl or methylene radical, with the proviso that 4-(2,2,3,6-tetramethyl-1-cyclohexyl)-2-butanone, 4-(2,2,3,6-tetramethyl-1-cyclohexyl)-3-buten-2-one, 1-(2,2,3,6-tetramethyl-1-cyclohexyl)-1-hexen-3-one and 1-(2,2,3,6-tetramethyl-1-cyclohexyl)-3-hexanone are excluded, said process being characterized in that :

a. a compound of formula

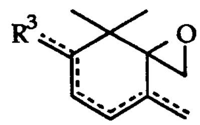

(II)

wherein the dotted lines and symbol $R^3$ are defined as above, is treated with a Lewis type acid to obtain an aldehyde of formula

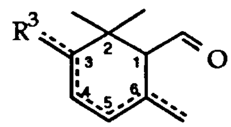

(III)

wherein the dotted lines and symbol $R^3$ have the meaning cited above ;

b. if necessary, an aldehyde of formula (III) having an endocyclic double bond in position 5 is converted into its isomer of formula

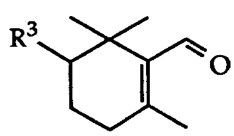

(IIIb)

wherein $R^3$ has the meaning indicated above, by means of a conventional isomerisation reaction ;

c. the aldehyde obtained according to a. or b. is converted into a compound of formula (I) having a double bond in the side chain, by means of an aldol condensation reaction with an appropriate ketone of formula

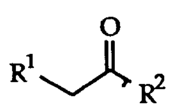

wherein $R^1$ and $R^2$ are defined as in formula (I) ; and

d. if necessary, the latter compound is subjected to a conventional hydrogenation or reduction reaction, to obtain a compound of formula (I) having a saturated side chain.

**15.** Process according to claim 14, characterized in that there is prepared a compound according to claim 1 by using as a starting product an epoxide of formula

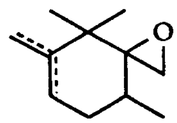

(IIa)

wherein the dotted lines have the meaning indicated in formula (Ia).

**16.** Process according to claim 15, characterized in that a compound according to claim 2 is prepared by using, as a starting product, an epoxide essentially in the form of a trans configuration isomer of formula

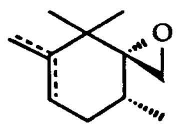

(IIb)

wherein the dotted lines are defined as in formula (Ib), or one of its optically active enantiomers.

**17.** Process according to claim 15, characterized in that a compound according to claim 3 is prepared by using, as a starting product, an epoxide essentially in a trans configuration isomeric form of formula

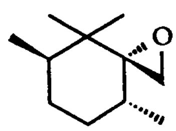

(IIc)

or one of its optically active enantiomers.

**18.** Process for the preparation of an aldehyde of formula (III) as defined in claim 14, excluding 2,2,3,6-tetramethyl-1-cyclohexanecarbaldehyde, characterized in that a compound of formula (II) as defined in claim 14 is treated with a Lewis acid.

**19.** Process according to claim 18, characterized in that a compound of formula (IIa) as defined in claim 15 is used as a starting product in order to obtain an aldehyde of formula

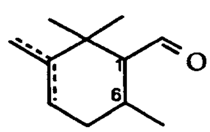

(IIIa)

wherein the dotted lines indicate the location of a double bond.

**20.** Compounds of formula (II) as defined in claim 14, excluding 4,4,5,8-tetramethyl-1-oxaspiro[2.5]octane.

**21.** Compound according to claim 20, in the form of a racemate of formula (IIb) as defined in claim 16, or of one of its optically active enantiomers.

**22.** Product according to claim 20, as a racemic stereoisomer of formula

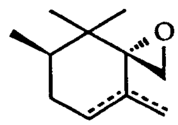

(IId)

wherein the dotted lines indicate the location of a double bond, or as one of its optically active enantiomers.

**23.** Compound of formula

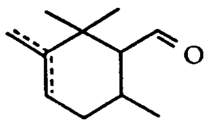

wherein the dotted lines indicate the location of a double bond.

**24.** Compound according to claim 23, as a racemic stereoisomer of formula

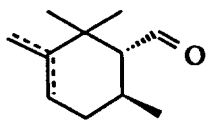

(IIIc)

wherein the dotted lines indicate the location of a double bond, or as one of its optically active enantiomers.

**Patentansprüche**

**1.** Verbindungen der Formel

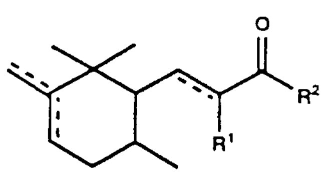

(Ia)

worin die punktierten Linien die Stelle einer Einfachbindung oder einer Doppelbindung angeben, das Symbol $R^1$ für ein Wasserstoffatom oder einen Methylrest steht, das Symbol $R^2$ einen gesättigten oder ungesättigten, geradekettigen oder verzweigten Alkylrest mit 1-4 Kohlenstoffatomen bedeutet, mit Ausnahme des 4-(2,2,3,6-Tetramethyl-1-cyclohexyl)-2-butanons, des 4-(2,2,3,6-Tetramethyl-1-cyclohexyl)-3-buten-2-ons, des 1-(2,2,3,6-Tetramethyl-1-cyclohexyl)-1-hexen-3-ons und des 1-(2,2,3,6-Tetramethyl-1-cyclohexyl)-3-hexanons.

2. Eine Verbindung gemäß Patentanspruch 1, im wesentlichen in Form eines Trans-Isomeren der Formel

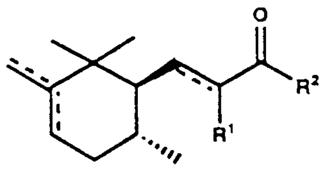

(Ib)

worin die punktierten Linien im Ring die Stelle einer Doppelbindung angeben, die punktierte Linie in der Seitenkette die Stelle einer Einfachbindung oder Doppelbindung angibt, das Symbol $R^1$ für ein Wasserstoffatom oder einen Methylrest steht, und das Symbol $R^2$ einen gesättigten oder ungesättigten, geradekettigen oder verzweigten Alkylrest mit 1-4 Kohlenstoffatomen bedeutet, in racemischer Form oder in Form eines optisch aktiven Enantiomeren.

3. Eine Verbindung gemäß Patentanspruch 1, im wesentlichen in Form eines Trans-Isomeren der Formel

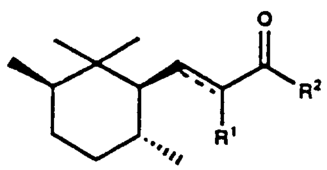

(Ic)

worin die punktierte Linie die Stelle einer Einfachbindung oder einer Doppelbindung angibt und die Symbole $R^1$ und $R^2$ die für Anspruch 1 angegebene Bedeutung besitzen, in Form eines Racemats oder eines optisch aktiven Enantiomeren, mit Ausnahme des 4-(2,2,c-3,t-6-Tetramethyl-r-1-cyclohexyl)-2-butanons, des 1-(2, 2,c-3,t-6-Tetramethyl-r-1-cyclohexyl)-1-hexen-3-ons und des 1-(2,2,c-3,t-6-Tetramethyl-r-1-cyclohexyl)-3-hexanons.

4. Als Verbindung gemäß Patentanspruch 2, eine der nachfolgenden Verbindungen:
   a. trans,(E)-4-(2,2,3,6-Tetramethyl-3-cyclohexen-1-yl)-3-buten-2-on
   b. trans,(E)-4-(2,2,6-Trimethyl-3-methylen-1-cyclohexyl)-3-buten-2-on
   c. trans,(E)-1-(2,2,6-Trimethyl-3-methylen-1-cyclohexyl)-1-penten-3-on

   in Form eines Racemats oder eines ihrer optisch aktiven Enantiomeren.

5. Als Verbindung gemäß Patentanspruch 3, das (E)-4-(2,2,c-3,t-6-Tetramethyl-r-1-cyclohexyl)-3-buten-2-on in Form eines Racemats oder eines seiner optisch aktiven Enantiomeren.

6. Verwendung einer Verbindung der Formel

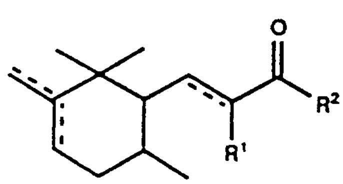

(Ia)

worin die punktierten Linien die Stelle einer Einfachbindung oder einer Doppelbindung angeben, das Symbol $R^1$ für ein Wasserstoffatom oder einen Methylrest steht, und das Symbol $R^2$ einen gesättigten oder ungesättigten, geradekettigen oder verzweigten Alkylrest mit 1-4 Kohlenstoffatomen bedeutet, als Riechstoffbestandteil für die Herstellung von Riechstoffkompositionen und parfümierten Artikeln.

7. Verwendung gemäß Patentanspruch 6, einer Verbindung gemäß Patentanspruch 2.

8. Verwendung gemäß Patentanspruch 6, einer Verbindung der Formel (Ic), wie sie im Patentanspruch 3 definiert wird, in Form eines Racemats oder eines ihrer optisch aktiven Enantiomeren.

**9.** Verwendung gemäß Patentanspruch 6, einer Verbindung gemäß Patentanspruch 4.

**10.** Verwendung gemäß Patentanspruch 6, einer Verbindung gemäß Patentanspruch 5.

**11.** Riechstoffkompositionen resultierend aus der Verwendung gemäß einem der Patentansprüche 6 bis 10.

**12.** Parfümierter Artikel resultierend aus der Verwendung gemäß einem der Patentansprüche 6 bis 10.

**13.** Als parfümierter Artikel gemäß Patentanspruch 12, ein Parfüm oder ein Toilettwasser, eine Seife, ein Dusch- oder Badegel, ein Shampoo oder ein anderes Produkt der Haarpflege, eine kosmetische Zusammensetzung, ein Körperdesodorant oder ein Raumluftverbesserer, ein Reinigungsmittel oder ein Textilauffrischungsmittel oder ein Pflegeprodukt.

**14.** Verfahren zur Herstellung einer Verbindung der Formel

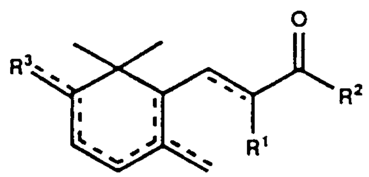

(I)

worin die punktierten Linien die Stelle einer Einfachbindung oder einer Doppelbindung angeben, das Symbol $R^1$ für ein Wasserstoffatom oder einen Methylrest steht, das Symbol $R^2$ einen gesättigten oder ungesättigten, geradekettigen oder verzweigten Alkylrest mit 1-4 Kohlenstoffatomen bedeutet, und das Symbol $R^3$ für einen Methyl-, Ethyl- oder Methylenrest steht, mit Ausnahme des 4-(2,2,3,6-Tetramethyl-1-cyclohexyl)-2-butanons, des 4-(2,2,3,6-Tetramethyl-1-cyclohexyl)-3-buten-2-ons, des 1-(2,2,3,6-Tetramethyl-1-cyclohexyl)-1-hexen-3-ons und des 1-(2,2,3,6-Tetramethyl-1-cyclohexyl)-3-hexanons, wobei das Verfahren dadurch gekennzeichnet ist, daß man

a. eine Verbindung der Formel

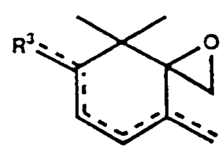

(II)

worin die punktierten Linien und das Symbol $R^3$ die obigen Bedeutungen besitzen, mit einer Lewis-Säure umsetzt, um ein Aldehyd der Formel

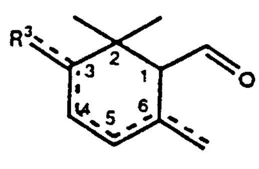

(III)

zu erhalten, worin die punktierten Linien und das Symbol $R^3$ die obigen Bedeutungen besitzen;

b. einen Aldehyd der Formel (III), der eine Ringdoppelbindung in Stellung 5 besitzt, gegebenenfalls mit Hilfe einer an sich bekannten Isomerisierungsreaktion in sein Isomeres der Formel

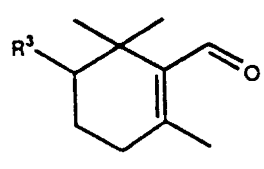

(IIIb)

worin das Symbol $R^3$ die oben angegebene Bedeutung besitzt, überführt;
c. den gemäß a. oder b. erhaltenen Aldehyd mit Hilfe einer Aldolkondensation mit einem geeigneten Keton der Formel

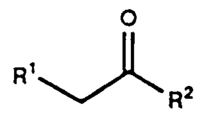

worin $R^1$ und $R^2$ die für Formel (I) angegebene Bedeutung besitzen, in eine Verbindung der Formel (I), die in der Seitenkette eine Doppelbindung besitzt, überführt; und
d. die letzgenannte Verbindung gegebenenfalls einer Hydrierungsreaktion oder einer an sich bekannten Reduktion unterwirft, um eine Verbindung der Formel (I) zu erhalten, die eine gesättigte Kette besitzt.

**15.** Verfahren gemäß Patentanspruch 14, dadurch gekennzeichnet, daß man eine Verbindung gemäß Patentanspruch 1 unter Verwendung eines Epoxids der Formel

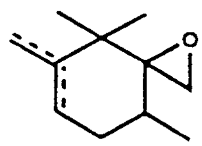

(IIa)

worin die punktierten Linien die für Formel (Ia) angegebene Bedeutung besitzen,als Ausgangsverbindung herstellt.

**16.** Verfahren gemäß Patentanspruch 15, dadurch gekennzeichnet, daß man eine Verbindung gemäß Patentanspruch 2 unter Verwendung eines Epoxids im wesentlichen in Form eines Transisomeren der Formel

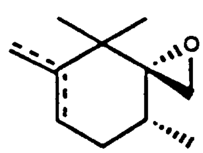

(IIb)

worin die punktierten Linien wie für Formel (Ib) definiert sind, oder eines seiner optisch aktiven Enantiomeren als Ausgangsverbindung herstellt.

**17.** Verfahren gemäß Patentanspruch 15, dadurch gekennzeichnet, daß man eine Verbindung gemäß Patentanspruch 3 unter Verwendung eines Epoxids im wesentlichen in Form eines Transisomeren der Formel

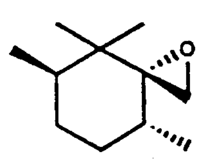

(IIc)

oder eines seiner optisch aktiven Enantiomeren als Ausgangsverbindung herstellt.

**18.** Verfahren zur Herstellung eines Aldehyds der Formel (III), wie er im Patentanspruch 14 definiert wird, mit Ausnahme des 2,2,3,6-Tetramethyl-1-cyclohexancarbaldehyds, dadurch gekennzeichnet, daß man eine Verbindung der Formel (II), wie sie im Patentanspruch 14 definiert wird, mit einer Lewis-Säure behandelt.

**19.** Verfahren gemäß Patentanspruch 18, dadurch gekennzeichnet, daß man eine Verbindung der Formel (IIa), wie sie im Patentanspruch 15 definiert wird, als Ausgangsverbindung verwendet, um einen Aldehyd der Formel

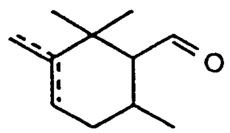

(IIIa)

worin die punktierten Linien die Stelle einer Doppelbindung angeben, zu erhalten.

**20.** Verbindung der Formel (II), wie sie im Patentanspruch 14 definiert wird, mit Ausnahme des 4,4,5,8-Tetramethyl-1-oxaspiro[2,5]oktans.

**21.** Verbindung gemäß Patentanspruch 20, in Form eines Racemats der Formel (IIb), wie es im Patentanspruch 16 definiert wird, oder eines ihrer optisch aktiven Enantiomeren.

**22.** Verbindung gemäß Patentanspruch 20, in Form der racemischen Stereoisomeren der Formel

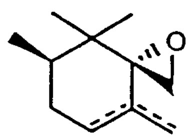

(IId)

worin die punktierten Linien die Stelle einer Doppelbindung angeben, oder in Form eines ihrer optisch aktiven Enantiomeren.

**23.** Verbindung der Formel

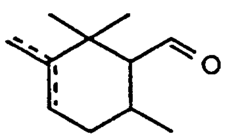

worin die punktierten Linien die Stelle einer Doppelbindung angeben.

**24.** Verbindung gemäß Patentanspruch 23, in Form der racemischen Stereoisomeren der Formel

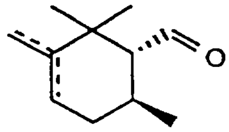

(IIIc)

worin die punktierten Linien die Stelle einer Doppelbindung angeben, oder in Form eines ihrer optisch aktiven Enantiomeren.